# EUROPÄISCHE PATENTSCHRIFT

(11) **EP 3 911 172 B1**
(45) Veröffentlichungstag und Bekanntmachung des Hinweises auf die Patenterteilung: **15.04.2026**
(21) Anmeldenummer: 19701211.5
(22) Anmeldetag: 18.01.2019
(51) Int. Cl.: A61K 31/11, A61K 31/165, A61K 31/198, A61K 36/48, A61P 43/00, A23L 2/52, A61K 38/01, A61K 35/00

(54) **KOMBINATION VON SÄTTIGUNGSKOMPONENTEN MIT ARGININ**
COMBINATION OF SATIETY COMPONENTS WITH ARGININE
COMBINAISON DE COMPOSANTS DE SATIÉTÉ AVEC DE L'ARGININE

(43) Veröffentlichungstag der Anmeldung: 24.11.2021
(73) Patentinhaber: Symrise AG, 37603 Holzminden (DE)
(72) Erfinder: HANS, Joachim, 37603 Holzminden (DE); LEY, Jakob, 37603 Holzminden (DE); SOMOZA, Veronika, 3400 Weidling (AT); STÖGER, Verena, 1110 Wien (AT)
(74) Vertreter: Global IP Europe Patentanwaltskanzlei
(86) Internationale Anmeldenummer: PCT/EP2019/051301
(87) Internationale Veröffentlichungsnummer: WO 2020/147977

(56) Entgegenhaltungen:
- EP-A1- 2 614 727
- EP-A1- 2 918 270
- WO-A1-2017/220168
- WO-A1-2018/129095
- WO-A2-2011/045732
- WO-A2-2011/063817
- US-A1- 2016 213 673
- CHRISTINA M. HOCHKOGLER ET AL: "A 12-week intervention with nonivamide, a TRPV1 agonist, prevents a dietary-induced body fat gain and increases peripheral serotonin in moderately overweight subjects", MOLECULAR NUTRITION & FOOD RESEARCH, vol. 61, no. 5, 22 February 2017 (2017-02-22), DE, pages 1600731, XP055634031, ISSN: 1613-4125, DOI: 10.1002/mnfr.201600731
- CHRISTINA M. HOCHKOGLER ET AL: "The capsaicin analog nonivamide decreases total energy intake from a standardized breakfast and enhances plasma serotonin levels in moderately overweight men after administered in an oral glucose tolerance test: A randomized, crossover trial", MOLECULAR NUTRITION & FOOD RESEARCH, vol. 58, no. 6, 1 June 2014 (2014-06-01), DE, pages 1282 - 1290, XP055633218, ISSN: 1613-4125, DOI: 10.1002/mnfr.201300821
- MUN JOO-MI ET AL: "Corn gluten hydrolysate and capsaicin have complimentary actions on body weight reduction and lipid-related genes in diet-induced obese rats", NUTRITION RESEARCH, vol. 34, no. 5, 1 May 2014 (2014-05-01), pages 458 - 465, XP028865381, ISSN: 0271-5317, DOI: 10.1016/J.NUTRES.2014.04.009
- SUSANA CAMACHO ET AL: "Anti-Obesity and Anti-Hyperglycemic Effects of Cinnamaldehyde via altered Ghrelin Secretion and Functional impact on Food Intake and Gastric Emptying", SCIENTIFIC REPORTS, vol. 5, no. 1, 21 January 2015 (2015-01-21), XP055633327, DOI: 10.1038/srep07919
- C. WANG ET AL: "Arginine affects appetite via nitric oxide in ducks", POULTRY SCIENCE, vol. 93, no. 8, 22 July 2014 (2014-07-22), Oxford, pages 2048 - 2053, XP055634102, ISSN: 0032-5791, DOI: 10.3382/ps.2013-03812

## Beschreibung

### Gebiet der Erfindung

Die vorliegende Erfindung betrifft primär ein Kombinationsmittel zur Reduzierung des Appetits und/oder zur Vermittlung eines Sättigungsgefühls und/oder zur Reduzierung der Energieaufnahme und/oder zur Reduzierung des Körpergewichts. Darüber hinaus betrifft die vorliegende Erfindung eine medizinische Verwendung des Kombinationsmittels, insbesondere zur Verwendung zur Behandlung von Übergewicht und Adipositas sowie zur Verwendung zur Prävention von kardiovaskulären Erkrankungen, Schlaganfall, Diabetes sowie Gelenkschäden und Verschleiß der Wirbelsäule, sowie eine nicht-therapeutische Verwendung des Kombinationsmittels. Der Fokus der vorliegenden Erfindung liegt insbesondere in der Verwendung des Kombinationsmittels, um ein Sättigungsgefühl zu vermitteln und/oder die Energieaufnahme zu reduzieren und/oder das Körpergewicht zu reduzieren. Schließlich betrifft die vorliegende Erfindung die Verwendung des Kombinationsmittels zur Herstellung einer oral konsumierbaren Zubereitung oder einer Lebensmittelzutat sowie eine daraus hergestellte oral konsumierbare Zubereitung oder Lebensmittelzutat.

### Stand der Technik

Der Mechanismus der Sättigung ist ein sehr komplexer körperlicher Vorgang, an dem zahlreiche Faktoren beteiligt sind, von denen noch immer nicht alle erforscht sind. Grundsätzlich dienen Hunger und Sättigung dazu, die menschliche Nahrungsaufnahme zu regulieren und die ausreichende Versorgung des Organismus sicherzustellen.

Bekannt ist, dass Hunger und Sättigung durch eine bestimmte Region im Gehirn, den Hypothalamus, vermittelt werden. Hier arbeiten zwei Zentren, Hungerzentrum und Sättigungszentrum. Diese Zentren regulieren eine Hormonabgabe ins Blut, die entweder Nahrungsaufnahme oder einen Essstopp veranlassen: Das Hungerzentrum induziert die Ausschüttung der "Hungerhormone" Neuropeptid Y, Agouti Related Peptide (AGRP) und Melanin Concentrating Hormone (MCH) sowie cannabisähnliche Hormone (Endocannaboide). Das Sättigungszentrum arbeitet vor allem mit den "Sättigungshormonen" Propiomelanocortin (Alpha-MSH), CART und Serotonin. Wie stark das Hungerzentrum und das Sättigungszentrum arbeiten, hängt von Signalen aus dem Körper ab: Ein abfallender Blutzuckerspiegel meldet Hunger, während das bei hohem Blutzuckerspiegel vermehrt ausgeschüttete Insulin das Sättigungszentrum aktiviert.

Auch Signale aus dem Magen-Darm-Trakt spielen eine Rolle: Durch die Dehnungsrezeptoren des Magens und Darms nach Füllung des Magens beim Essen werden afferente Signale an das zentrale Nervensystem weitergeleitet. Zusätzlich werden bestimmte Hormone im Magen und Darm (wie etwa Leptin, Cholecystokinin oder das Glucagon Like Peptide 1) ausgeschüttet und stimulieren das Sättigungszentrum. Des Weiteren zeigen neuere Studien, dass auch ein erhöhtes Level an peripher vorkommendem Serotonin, das von enterochromaffinen Zellen im Darm nach Stimulierung ausgeschüttet wird, mit einem verstärkten Sättigungsgefühl verbunden ist (Hochkogler, Christina M. et al., The capsaicin analog nonivamide decreases total energy intake from a standardized breakfast and enhances plasma serotonin levels in moderately overweight men after administered in an oral glucose tolerance test: A randomized, crossover trial, Molecular nutrition & food research, 58.6 (2014): 1282 - 1290). Umgekehrt wurden niedrigere Serotonin-Level im Plasma mit verstärktem Appetit assoziiert (Hochkogler, Christina M. et al., Appetite-inducing effects of homoeriodictyol: Two randomized, cross-over interventions, Molecular nutrition & food research, 61.12 (2017): 1700459).

Das durch mangelnde Bewegung und/oder zu hohe Nahrungsaufnahme und/oder Störungen des Sättigungsmechanismus, wie beispielsweise Adipositas, einer Ernährungs- und Stoffwechselkrankheit, und Essstörungen, wie beispielsweise Binge Eating (Heißhungerattacken), verursachte häufige Auftreten von andauerndem Übergewicht kann zu Folgeerkrankungen wie Insulinresistenz, Lipidstoffwechselstörungen, Bluthochdruck, Diabetes Typ II, Arteriosklerose, Herz- oder Hirninfarkt und damit letztendlich zum verfrühten Tod führen. Insbesondere ein hoher Gehalt an leicht verstoffwechselbaren Kohlenhydraten, Proteinen und vor allem Fetten in der Nahrung führt zur Bildung von Fettdepots und kann schließlich zu den oben genannten Problemen stark beitragen.

Um einer erhöhten Aufnahme kalorisch relevanter Nahrungsmittelbestandteile entgegenzuwirken, besteht schon länger der Wunsch, orale Zubereitungen bereitzustellen, die das Hungergefühl und den natürlichen Appetit reduzieren und/oder das Sättigungsgefühl entsprechend erhöhen können.

Um die Aufnahme der hedonisch oft sehr bevorzugten Nahrungsmittelbestandteile, vor allem der Fette und süßen Kohlenhydrate (Zucker), zu begrenzen, werden deren Gehalte in kalorienreduzierten Lebensmitteln, den sogenannten "Light-Produkten", stark gesenkt und diese oft durch Ersatzstoffe (Verdicker für Fette, nicht-kalorische Süßstoffe anstelle der Zucker) ausgetauscht.

Beim Konsum von "Light-Produkten" kann es allerdings bei einem Produkt, das aufgrund geschickter Formulierung einen vergleichbaren hedonischen Wert wie das hoch-energetische Originalprodukt besitzt, vorkommen, dass es in größeren Mengen konsumiert wird und es dann im ungünstigsten Fall sogar zu einer erhöhten Aufnahme kalorisch relevanter Nahrungsmittelbestandteile kommt. In solchen Fällen wird das Ziel verfehlt, die aufgenommene Kalorienmenge abzusenken.

Bei der Behandlung von Übergewicht und Adipositas werden verschiedene Methoden eingesetzt, um die Nahrungsaufnahme der Betroffenen zu begrenzen. Einen moderaten Effekt haben Ballaststoffe mit hohem Wasserbindungsvermögen, wie Glucomannane (Konjak) oder Flohsamenschalen.

Daneben gibt es auf dem Markt unterschiedliche Anorektika (Appetitzügler, auch Appetithemmer). Anorektika sind Arzneistoffe mit einer Appetit-hemmenden Wirkung. Diese Wirkung beruht auf einer Hemmung des Hungerzentrums oder einer Beeinflussung des Sättigungszentrums im Hypothalamus des Gehirns. Die bekanntesten Vertreter dieser Stoffgruppe sind Aminorex, Cathin, Ephedrin, Phentermin, Phenylpropanolamin (PPA) bzw. Norephedrin, Fenfluramin, Sibutramin, Nikotin und Rimonabant. Viele dieser Arzneistoffe werden nicht mehr therapeutisch verwendet, da die längere Einnahme zur Abhängigkeit führt und schwere Nebenwirkungen auslösen kann.

Es ist bekannt, dass durch Erhöhung der Dopamin- und der Serotonin-Ausschüttung in bestimmten Hirnarealen bei gleichzeitiger Exposition mit Nährstoffen durch die aufgenommenen oral konsumierbaren Produkte (insbesondere Lebensmittel) sowie durch Induktion von Leptin-Rezeptor- und Serotonin-Rezeptor-Proteinen der Appetit negativ und die Sättigung positiv beeinflusst werden können, beispielsweise durch Nonivamid. Neben einer Einwirkung auf den Appetit und auf die Sättigung kann damit auch gleichzeitig die Stimmung des Konsumenten positiv beeinflusst werden. So sind bereits Lebensmittel bekannt, die Dopamin und Serotonin umfassen und eine stimmungsaufhellende Wirkung besitzen.

In Untersuchungen hat sich gezeigt, dass reines Capsaicin, der wichtigste Scharfstoff aus der Chillischote (*Capsicum anuum*), einen Appetit-reduzierenden und Sättigungs-steigernden Effekt zeigen kann (Smeets, A. J. P. G., Westerterp-Plantenga, M., Capsaicin, in weight control and slimming ingredients in Food Technologies, Cho, Susan S. (Ed.), 201 - 211, Wiley-Blackwell: Ames, lowa, 2010). Dieser beobachtete Effekt beruht vermutlich darauf, dass Capsaicin bei oraler Aufnahme ein Gefühl von Hitze vermittelt.

Der Einsatz von Capsaicin in Lebensmitteln ist allerdings in der Europäischen Union nicht erlaubt (wurde von der Community Flavoring List in 2004 gestrichen), da das genotoxische Potential der Verbindung negativ bewertet wurde (European Food Safety Authority (EFSA), P., Italy, Opinion of the Scientific Committee on Food on Capsaicin. European Comission 2002, (SDF/CS/FLAV/FLAVOUR/8 ADD1 Final)). Zudem ist der Einsatz in Lebensmitteln oft schwierig, da Capsaicin einen niedrigen Geschmacksschwellenwert (sensorischer Erkennungsschwellenwert) und eine hohe Potenz als Scharfstoff (16,000,000 Scoville units, vgl. http://en.wikipedia.org/wiki/Capsaicin; Version des Eintrags mit letzten Änderungen vom 11. November 2011, 21:02 Uhr) hat. Capsaicin wird, auch auf Grund des hohen Preises des Reinstoffs, zudem nahezu ausschließlich in Form eines Capsicumextrakts verwendet, der neben weiteren Scharfstoffen noch Reste anderer, nach Capsicum schmeckender oder riechender Aromastoffe enthält und daher für den breiten Einsatz nur bedingt geeignet ist.

In der EP 2 614 727 A1 wird Nonivamid (N-Nonanoylvanillylamin) als äußerst wirksamer Aromastoff beschrieben, der die oben beschriebenen gewünschten Wirkungen, nämlich Appetit-reduzierend und Sättigungs-steigernd, zeigt. Allerdings ist durch die intrinsische Schärfe des Stoffes die Einsetzbarkeit, insbesondere in milden aromatischen Endanwendungen, problematisch.

Die WO 2017/220168 A1 offenbart alternativ nicht scharf schmeckende Cinnamylalkohol-Derivate zur Anwendung in einem therapeutischen und nicht-therapeutischen Verfahren als Mittel zur Reduzierung des Appetits, zur Vermittlung eines Gefühls von Sättigung, zur Reduzierung der Energieaufnahme, zur Reduzierung des Körpergewichts oder als Stimmungsaufheller. Allerdings sind diese Derivate nicht so effektiv wie Nonivamid und müssen daher relativ hoch dosiert werden.

In der EP 2 918 270 A1 werden im Vergleich zu Nonivamid weniger scharf schmeckende Alkensäure-Derivate zur Anwendung in einem therapeutischen und nicht-therapeutischen Verfahren als Mittel zur Reduzierung des Appetits, zur Vermittlung eines Gefühls von Sättigung, zur Reduzierung der Energieaufnahme oder als Stimmungsaufheller vorgeschlagen. Die Stoffe sind aber derzeit kaum kommerziell verfügbar und auch aus natürlichen Extrakten nicht in ausreichenden Mengen isolierbar. Die Wirksamkeit der genannten Stoffe ist im Vergleich zu Nonivamid ebenfalls geringer, was eine höhere Dosierung mit entsprechenden sensorischen Effekten bedeutet.

Primäre Aufgabe der vorliegenden Erfindung ist deshalb, ein Kombinationsmittel, insbesondere in Bezug auf die bekannten Effekte des Nonivamids, bereitzustellen, das in seiner Wirkung als Mittel zur Reduzierung des Appetits und/oder als Mittel zur Vermittlung eines Gefühls von Sättigung und/oder als Mittel zur Reduzierung der Energieaufnahme und/oder als Mittel zur Reduzierung des Körpergewichts verwendet werden kann und dabei insbesondere deutlich stärkere Effekte bei gleicher oder geringerer Dosierung im Vergleich zu beispielsweise Nonivamid hervorruft.

Ferner war es Aufgabe der vorliegenden Erfindung, ein solches Kombinationsmittel, insbesondere ein oral konsumierbares Produkt, bereitzustellen, das bereits in geringen Mengen die oben genannten Wirkungen zeigt und das beim Verzehr keine unangenehmen sensorischen Noten oder einen Nebengeschmack verursacht oder so eingesetzt werden kann, dass es die erfindungsgemäße Aufgabe löst und dabei die organoleptischen Eigenschaften eines oral konsumierbaren Produktes (insbesondere eines Lebensmittels, Futtermittels oder Arzneimittels), in dem es eingesetzt werden kann, nicht bzw. nicht signifikant beeinflusst.

### Zusammenfassung der Erfindung

Die vorliegenden Problemstellungen werden durch die Gegenstände der unabhängigen Patentansprüche gelöst.

Ein erster Gegenstand der vorliegenden Erfindung betrifft ein Kombinationsmittel, umfassend oder bestehend aus den folgenden Bestandteilen:
(a) eine oder mehrere erste Sättigungskomponente(n), wobei die eine oder die mehreren erste(n) Sättigungskomponente(n) ausgewählt ist/sind aus der Gruppe, bestehend aus Nonivamid, Pellitorin, Cinnamylalkohol-Derivaten, ausgewählt aus der Gruppe, bestehend aus Cinnamylformiat, Cinnamylacetat, Cinnamylpropionat, Cinnamylbutyrat, Cinnamylisobutyrat, Cinnamylisovalerianat, Cinnamyltiglinat, Cinnamylbenzoat, Cinnamylphenylacetat, und Cinnamylcinnamat, Zimtsäureamid-Derivaten der allgemeinen Formel 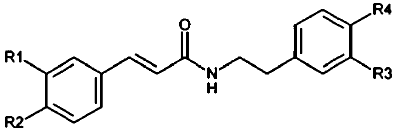 wobei R1, R2, R3 und R4 unabhängig voneinander für Wasserstoff oder eine Methoxygruppe stehen, aromatischen Alkensäure-Derivaten, ausgewählt aus der Gruppe, bestehend aus Dihydropiperlinguminin, Chingchengenamid A, Piperdardin und Methyl-7-(1,3-benzodioxol-5-yl)hepta-2,4-dienoat, und Mischungen daraus;;
(b) zugesetztes freies Arginin oder dessen möglichen Stereoisomeren und Mischungen der Stereoisomeren oder dessen Natrium-, Calcium-, Magnesium-, Kalium- oder Ammoniumsalzen; und
   optional
(c) eine oder mehrere zweite Sättigungskomponente(n), wobei die eine oder die mehreren zweite(n) Sättigungskomponente(n) ausgewählt ist aus der Gruppe, bestehend aus pflanzlichen oder tierischen Proteinen und Proteinhydrolysat(en); und/oder
(d) ein oder mehrere sensorisch aktive(s) Additiv(e).

Eine zweite erfindungsgemäße Ausgestaltung betrifft die medizinische oder die nicht-medizinische Verwendung des Kombinationsmittels.

Des Weiteren betrifft die vorliegende Erfindung die Verwendung des o.g. Kombinationsmittels zur Verwendung als Mittel zur
(i) Reduzierung des Appetits; und/oder
(ii) Vermittlung eines Sättigungsgefühls; und/oder
(iii) Reduzierung der Energieaufnahme; und/oder
(iv) Reduzierung des Körpergewichts.

Letztlich betrifft die vorliegende Erfindung die Verwendung des Kombinationsmittel zur Herstellung einer oral konsumierbaren Zubereitung, insbesondere eines Lebensmittels, eines Nahrungsergänzungsmittels, eines Futtermittels und eines Arzneimittels, oder einer Lebensmittelzutat sowie eine oral konsumierbare Zubereitung oder Lebensmittelzutat, welche das erfindungsgemäße Kombinationsmittel umfasst.

Weitere Aspekte der vorliegenden Erfindung und bevorzugte Ausgestaltungen ergeben sich aus der nachfolgenden detaillierten Beschreibung sowie insbesondere aus dem Wortlaut der beigefügten Patentansprüche.

### Figuren

Figur 1 ist ein Diagramm, welches das subjektive Hungergefühl vor und nach Verabreichung von erfindungsgemäßen Kombinationsmitteln (Kontrolle als Linie dargestellt) zeigt.
Figur 2 ist ein Diagramm, welches die *ad libitum* Energieaufnahme 140 min nach Verabreichung von erfindungsgemäßen Kombinationsmitteln (Kontrolle als Linie dargestellt) zeigt.
Figur 3 ist ein Diagramm, welches die Hungerhormon Ghrelin-Konzentrationen 15, 30, 60, 90 und 120 min nach Verabreichung von erfindungsgemäßen Kombinationsmitteln zeigt.
Figur 4 ist eine Korrelationsanalyse zwischen den Δ AUC (Fläche unter der Kurve) Werten von Serotonin und der Magenentleerung.

### Detaillierte Beschreibung der Erfindung

In einem ersten Aspekt betrifft die vorliegende Erfindung ein Kombinationsmittel, umfassend oder bestehend aus den folgenden Bestandteilen:
(a) eine oder mehrere erste Sättigungskomponente(n),
   wobei die eine oder die mehreren erste(n) Sättigungskomponente(n) ausgewählt ist/sind aus der Gruppe, bestehend aus Nonivamid, Pellitorin, Cinnamylalkohol-Derivaten, ausgewählt aus der Gruppe, bestehend aus Cinnamylformiat, Cinnamylacetat, Cinnamylpropionat, Cinnamylbutyrat, Cinnamylisobutyrat, Cinnamylisovalerianat, Cinnamyltiglinat, Cinnamylbenzoat, Cinnamylphenylacetat, und Cinnamylcinnamat, Zimtsäureamid-Derivaten der allgemeinen Formel 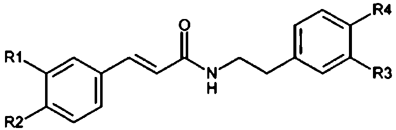
   wobei R1, R2, R3 und R4 unabhängig voneinander für Wasserstoff oder eine Methoxygruppe stehen, aromatischen Alkensäure-Derivaten, ausgewählt aus der Gruppe, bestehend aus Dihydropiperlinguminin, Chingchengenamid A, Piperdardin und Methyl-7-(1,3-benzodioxol-5-yl)hepta-2,4-dienoat, und Mischungen daraus;
(b) zugesetztes freies Arginin oder dessen möglichen Stereoisomeren und Mischungen der Stereoisomeren oder dessen Natrium-, Calcium-, Magnesium-, Kalium- oder Ammoniumsalzen; und
   optional
(c) eine oder mehrere zweite Sättigungskomponente(n), wobei die eine oder die mehreren zweite(n) Sättigungskomponente(n) ausgewählt ist aus der Gruppe, bestehend aus insbesondere pflanzlichen oder tierischen Proteinen und ein Protein oder ein Proteinhydrolysat(en) ; und/oder
(d) ein oder mehrere sensorisch aktive(s) Additiv(e).

Die vorliegende Erfindung beruht auf der überraschenden Erkenntnis, dass sich mit dem erfindungsgemäßen Kombinationsmittel, insbesondere einem oral konsumierbaren Produkt, der Appetit reduzieren und ein schnelleres Sättigungsgefühl vermitteln lässt, wodurch gleichzeitig die Energieaufnahme verringert wird, und/oder sich damit der Grundumsatz steigern lässt. Die verringerte Energieaufnahme und/oder Steigerung des Grundumsatzes führen letztendlich zu einer Gewichtsreduktion.

Im Folgenden werden die Bestandteile (a) bis (d) des erfindungsgemäßen Kombinationsmittels gemäß dem ersten Aspekt der vorliegenden Erfindung sowie deren bevorzugte Ausgestaltungen beschrieben und sind untereinander, soweit technisch sinnvoll, beliebig kombinierbar.

Bestandteil (a) - eine oder mehrere erste Sättigungskomponente(n)

Bei der einen oder den mehreren erste Sättigungskomponente(n) des erfindungsgemäßen Kombinationsmittels handelt es sich um Stoffe mit einem sensorischen Geschmacks- oder Geruchswert (Aromastoffe) mit gleichzeitig gewichtsreduzierender Wirkung, wobei die gewichtsreduzierende Wirkung hauptsächlich auf einer Hemmung des Hungerzentrums oder einer Beeinflussung des Sättigungszentrums im Hypothalamus des Gehirns beruht. Ferner können diese Stoffe mit einer sympathomimetischen Wirkung durch Steigerung des Grundumsatzes zur Gewichtsreduktion beitragen. Die Wirkung kann aber bevorzugt auch auf Erhöhung des peripheren Plasma-Serotonin-Spiegels beruhen. Umgekehrt wurden niedrigere Serotonin-Level im Plasma mit verstärktem Appetit assoziiert (Hochkogler, Christina M. et al., Appetite-inducing effects of homoeriodictyol: Two randomized, cross-over interventions, Molecular nutrition & food research, 61.12 (2017): 1700459).

Derartige in dem erfindungsgemäßen Kombinationsmittel zum Einsatz kommende erste Sättigungskomponenten sind im vorliegenden Kontext bevorzugt natürlich vorkommende niedermolekulare Stoffe wie z.B. Nonivamid, trans-Pellitorin, Cinnamylalkohol-Derivate, Zimtsäureamid-Derivate oder aromatische Alkensäure-Derivate, die den peripheren Serotoninspiegel erhöhen. Diese Sättigungskomponenten beeinflussen dadurch auch den Lipid- und/oder Glucosestoffwechsel, die Magenverweilzeit des Bolus und verringern letztendlich das Hungergefühl und/oder sorgen für ein lang-anhaltendes Sättigungsgefühl.

Den erste Sättigungskomponenten gemeinsam ist, dass sie eine sensorisch aktive Wirkung aufweisen, In dem erfindungsgemäßen Kombinationsmittel werden die eine oder die mehreren erste Sättigungskomponente(n) allerdings in einer Menge unterhalb oder in der Nähe ihres sensorischen Erkennungsschwellenwertes (niedrigste Konzentration, bei der ein Geschmacks- oder Geruchseindruck wahrnehmbar ist) und somit nicht in aromagebenden Konzentrationen eingesetzt, so dass beim Verzehr des erfindungsgemäßen Kombinationsmittels keine unangenehmen sensorischen Noten oder ein unerwünschter Nebengeschmack auftreten. Gleichzeitig werden jedoch in dem eingesetzten Konzentrationsbereich die oben beschriebenen und erwünschten physiologischen Effekte, nämlich Appetit-reduzierend und Sättigungs-steigernd, erzielt.

Bei allen Ausführungsformen der erste Sättigungskomponente ist die erste Sättigungskomponente oder eine Mischung aus mehreren der erste Sättigungskomponenten bevorzugt, die nur einen unterschwelligen Geruchs- oder Geschmackswert in der Einsatzkonzentration zeigt, d.h. bevorzugt wird die erste Sättigungskomponente oder eine Mischung aus mehreren der erste Sättigungskomponenten um den Erkennungsschwellenwert herum oder niedriger eingesetzt.

In einer bevorzugten Ausführungsform der vorliegenden Erfindung ist die erste Sättigungskomponente Nonivamid.

### Nonivamid:

Nonivamid bzw. N-Nonanoylvanillylamin (wie beschrieben in EP 2 614 727 A1, die bezüglich der darin offenbarten entsprechenden Verbindungen auf dem Wege der Verweisung Bestandteil dieser Anmeldung wird) ist ein bekannter und zugelassener Aromastoff (Flavis-Nummer 16.006; EFSA: ist in FGE.86 mit 'no safety concern' bewertet worden; CAS-Nummer 2444-46-4) und wird bereits in einer Vielzahl von Lebensmitteln zur Erzielung einer deutlichen Schärfe eingesetzt. Der Reizschwellenwert von N-Nonanoylvanillylamin liegt bei 0,054 mg/kg, der Erkennungsschwellenwert 0,107 mg/kg (vgl. Kollmannsberger, H., Inhaltsstoffzusammensetzung und sensorische Qualität von 20 Kultivaren verschiedener Capsicum-Arten, Dissertation, Technische Universität München, Weihenstephan, 2007, Seite 9).

N-Nonanoylvanillylamin ist ebenfalls unter den Bezeichnungen Pseudocapsaicin, N-((4-Hydroxy- 3-methoxyphenyl-) methyl-)nonanamid, Pelargonsäure-Vanillylamid oder Nonivamid bekannt. N-Nonanoylvanillylamin (Verbindung I) ist zur Verdeutlichung in der folgenden Abbildung dargestellt:

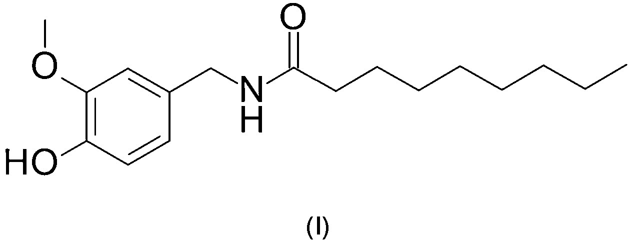

Wie vorstehend bereits am Beispiel des Capsaicins angemerkt, ist die Verwendung scharf schmeckender Verbindungen nicht immer problemlos möglich. Häufig ist eine Schärfe von oral konsumierbaren Produkten (insbesondere Lebensmittel, Futtermittel und Arzneimittel) nicht erwünscht, und zu scharf schmeckende oral konsumierbare Produkte werden vom Konsumenten häufig abgelehnt.

Überraschenderweise hat sich gezeigt, dass N-Nonanoylvanillylamin in der Lage ist, bereits
- in einem Konzentrationsbereich von 0,01 µM bis 10 µM (0,003 mg/kg bis 3 mg/kg) die Serotonin-Freisetzung in Neuronen um bis zu ca. 270 % gegen die jeweilige Kontrolle anzuregen
   und
- in einer Konzentration von etwa 0,03 mg/kg (entspricht 0,1 µM) die Dopamin-Freisetzung nach Acetylcholin-Stimulierung um bis zu ca. 700 % gegen die jeweilige Kontrolle anzuregen,
also jeweils in einer Konzentration, die sowohl deutlich unterhalb der Schwellenwerte als auch weit unter typischen Einsatzkonzentrationen in oral konsumierbaren Produkten (insbesondere Lebensmitteln, Futtermitteln oder Arzneimitteln) liegt.

Die Lipophilie des N-Nonanoylvanillylamins bedingt generell eine gute Bioverfügbarkeit, d.h. eine gute Resorption vom Magen-Darm-Trakt in die Blutlaufbahn. So treten insbesondere auch bei Einsatzkonzentrationen des N-Nonanoylvanillylamins unterhalb des geschmacklichen Erkennungsschwellenwerts, also unterhalb von 0,1 mg/kg, und bei Aufnahme üblicher Verzehrmengen von oral konsumierbaren Produkten (insbesondere von Lebensmitteln, Futtermitteln und Arzneimitteln) die oben genannten biologischen Effekte *in vivo* ein.

Vorzugsweise wird N-Nonanoylvanillylamin in dem Kombinationsmittel in einer Konzentration von 1 mg/kg oder weniger verwendet, bezogen auf das Gesamtgewicht des Kombinationsmittels, vorzugsweise in einer Konzentration von 0,5 mg/kg oder weniger, besonders bevorzugt in einer Konzentration von 0,1 mg/kg oder weniger, ganz besonders bevorzugt in einer Konzentration von 0,05 mg/kg oder weniger.

In einer weiteren Variante des Kombinationsmittels gemäß dem ersten Aspekt der vorliegenden Erfindung ist die eine oder sind die mehreren erste Sättigungskomponente(n) ausgewählt aus der Gruppe, die besteht aus: trans-Pellitorin, Cinnamylalkohol-Derivaten, Zimtsäureamid-Derivaten und aromatischen Alkensäure-Derivaten und Mischungen daraus. Diese können vorzugsweise auch in Kombination mit Nonivamid (N-Nonanoylvanillylamin) eingesetzt werden, was zu vorteilhaften Synergien in den Effekten führt.

### Pellitorin:

Pellitorin (trans-Pellitorin) ist ein Hauptwirkstoff der Bertramwurzel. Es zeigt einen stark betäubenden, als "Tingling" bezeichneten, sensorischen Effekt und regt stark den Speichelfluss an. In kleinen Mengen wird Pellitorin als Aromastoff zur Maskierung von Adstringentien oder zur Erhöhung der Saftigkeit eingesetzt. Pellitorin (Verbindung III) ist zur Verdeutlichung in der folgenden Abbildung dargestellt:

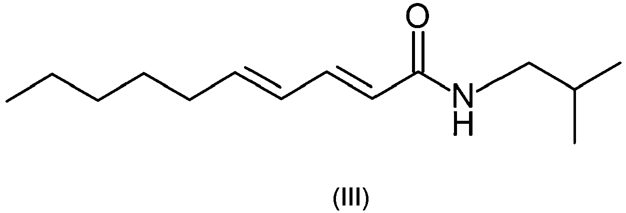

### Der Erkennungsschwellenwert von Pellitorin liegt bei 0,1 mg/kg.

Pellitorin wird in dem Kombinationsmittel gemäß der vorliegenden Erfindung in einer Konzentration von 2 mg/kg oder weniger, bezogen auf das Gesamtgewicht des Kombinationsmittels, verwendet, vorzugsweise in einer Konzentration von 0,2 mg/kg oder weniger, besonders bevorzugt in einer Konzentration von 0,1 mg/kg oder weniger. Vorzugsweise wird Pellitorin, bezogen auf das Gesamtgewicht Kombinationsmittels, in einer Konzentration von zumindest 0,0001 mg/kg oder mehr, vorzugsweise in einer Konzentration von 0,001 mg/kg oder mehr und ganz besonders bevorzugt in einer Konzentration von 0,005 mg/kg oder mehr eingesetzt.

### Cinnamylalkohol-Derivate:

Eine weitere, alternativ in dem Kombinationsmittel gemäß der vorliegenden Erfindung verwendete erste Sättigungskomponente ist ein Cinnamylalkohol-Derivat der Formel (V) (wie beschrieben in WO 2017/220168 A1, die bezüglich der darin offenbarten entsprechenden Verbindungen auf dem Wege der Verweisung Bestandteil dieser Anmeldung wird) oder ist eine Mischung aus zwei oder mehr Cinnamylalkohol-Derivaten der Formel (V): 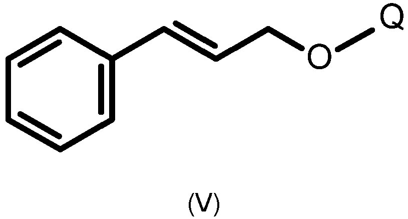 wobei die Doppelbindung eine E- oder Z-Konfiguration aufweisen kann oder eine beliebige Mischung von E- und Z-Konfigurationen vorliegen kann, und wobei Q r ein Acylrest der Formel (VI) 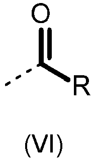 ist, wobei R Wasserstoff oder Methyl oder ein gesättigter oder ungesättigter, linearer oder verzweigter oder cyclischer aliphatischer Rest mit 2 bis 6 Kohlenstoffatomen oder Phenyl oder Phenylmethyl ist.

Gemäß einer bevorzugten Ausführungsform ist bei dem Cinnamylalkohol-Derivat der Formel (V) oder der Mischung aus zwei oder mehr Cinnamylalkohol-Derivaten der Formel (V) R (im Falle des Vorliegens einer Mischung jeweils unabhängig voneinander) ausgewählt aus der Gruppe bestehend aus Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl, 3-Methyl-3-pentyl, Vinyl, E- und Z- 1-Propen-1-yl, 2-Propen-1-yl, E- und Z- 2-Buten-2-yl, 3-Buten-2-yl, E- und Z-Prenyl, E- und Z-Isoprenyl, Phenyl und Phenylmethyl.

Insbesondere erfindungsgemäß bevorzugt ist das Cinnamylalkohol-Derivat der Formel (V) ausgewählt aus der Gruppe, die besteht aus: 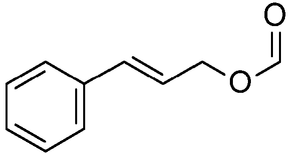 Cinnamylformiat (Verbindung 2, FEMA# 2299) 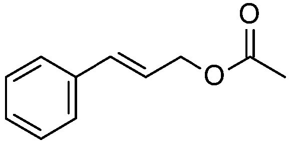 Cinnamylacetat (Verbindung 3, FEMA# 2293) 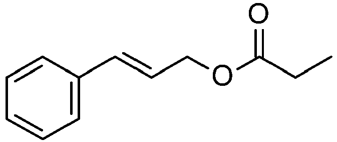 Cinnamylpropionat (Verbindung 4, FEMA# 2301) 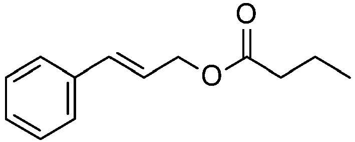 Cinnamylbutyrat (Verbindung 5, FEMA# 2296) 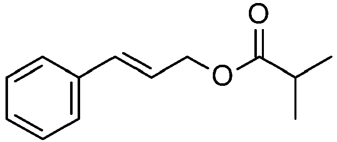 Cinnamylisobutyrat (Verbindung 6, FEMA# 2297) 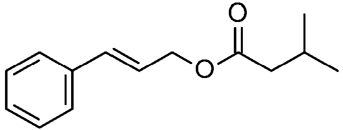 Cinnamylisovalerianat (Verbindung 7, FEMA# 2302) 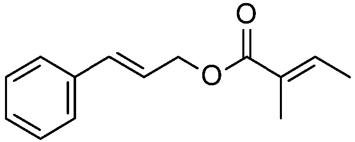 Cinnamyltiglinat (Verbindung 8, EC Aromastoffliste 09.339) 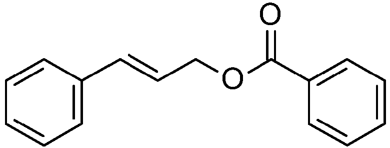 Cinnamylbenzoat (Verbindung 9, FEMA# 4703) 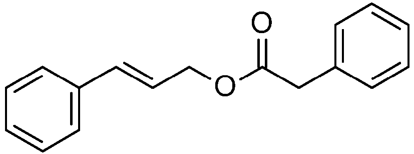 Cinnamylphenylacetat (Verbindung 10, FEMA# 2300) und 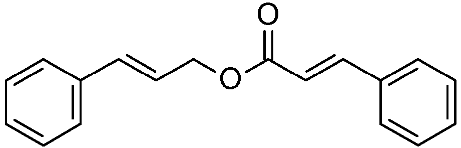 Cinnamylcinnamat (Verbindung 11, FEMA# 2298)

Gemäß einer weiteren bevorzugten Ausführungsform der vorliegenden Erfindung liegt das Cinnamylalkohol-Derivat der Formel (V) oder liegen die Cinnamylalkohol-Derivate der Formel (V) in der Mischung (jeweils) zu mehr als 50 %, bevorzugt zu mehr als 90 %, besonders bevorzugt zu mehr als 95 % in der E-Konfiguration vor.

Das Kombinationsmittel gemäß dem ersten Aspekt der vorliegenden Erfindung umfasst vorzugsweise ein Cinnamylalkohol-Derivat der Formel (V) oder eine Mischung aus mindestens zwei, drei, vier, fünf, sechs, sieben, acht, neun, zehn oder elf Cinnamylalkohol-Derivaten der Formel (V), welche bevorzugt ausgewählt sind aus der Gruppe der Verbindungen, bestehend aus: Verbindung 2, Verbindung 3, Verbindung 4, Verbindung 5, Verbindung 6, Verbindung 7, Verbindung 8, Verbindung 9, Verbindung 10 oder Verbindung 11. Ebenfalls ist es vorteilhaft, wenn alle 11 Verbindungen in einem solchen Kombinationsmittel vorliegen. Bevorzugt sind hierbei Verbindungen 1 und 2.

Die erfindungsgemäß anzuwendenden Cinnamylalkohol-Derivate der Formel (V) sind in der Lage, bereits in einem Konzentrationsbereich von 10 µM (ca. 1 mg/kg bis ca. 3 mg/kg) die Serotonin-Freisetzung in Neuronen auf bis zu ca. 130 % der Positivkontrolle (Nonivamid, EP 2 614 727 A1) anzuregen. Die jeweiligen Konzentrationen der aromatischen Cinnamylalkohol-Derivate sind damit im Vergleich zu typischen Einsatzkonzentrationen, um einen Geschmackseffekt zu erzielen, gering. Es ist zwar mit Aromaeffekten zu rechnen, diese sollten aber auch in mild aromatisierten oral konsumierbaren Produkten nicht bestimmend sein.

Gemäß einer bevorzugten Ausgestaltung liegt die Gesamtkonzentration der Cinnamylalkohol-Derivate der Formel (V) in dem Kombinationsmittel bei 20 mg/kg oder weniger, bevorzugt bei 5 mg/kg oder weniger, besonders bevorzugt bei 2 mg/kg oder weniger, jeweils bezogen auf das Gesamtgewicht des Kombinationsmittels. Bei der Bestimmung der Gesamtkonzentration der Cinnamylalkohol-Derivate in der Mischung ist die Summe der Konzentrationen der einzelnen Verbindungen gemeint.

### Zimtsäuream id-Derivate:

In einer weiteren Variante der vorliegenden Erfindung ist die eine erste Sättigungskomponente ein Zimtsäureamid-Derivat der Formel (VII) (wie beschrieben in EP 2 737 807 A1, die bezüglich der darin offenbarten entsprechenden Verbindungen auf dem Wege der Verweisung Bestandteil dieser Anmeldung wird) oder eine Mischung aus zwei oder mehreren Zimtsäureamid-Derivaten der Formel (VII): 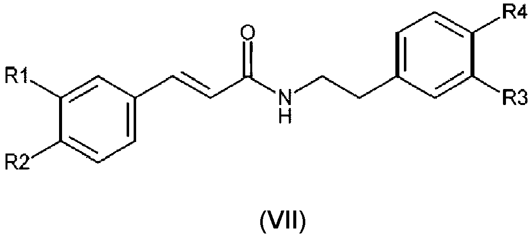 wobei R1, R2, R3und R4 unabhängig voneinander für Wasserstoff oder eine Methoxygruppe stehen.

Unter den erfindungsgemäß zu verwendenden Zimtsäureamid-Derivaten haben sich solche als besonders vorteilhaft erwiesen, bei denen in Formel (VII) mindestens eine der Gruppen R1, R2, R3 und R4 in der Formel (VI) für Wasserstoff steht. Ganz besonders bevorzugt ist der Einsatz von Zimtsäureamid-Derivaten, die denen in der Formel (VII) R1, R2 und R4 für eine Methoxygruppe und R3 für Wasserstoff steht.

Das Kombinationsmittel gemäß dem ersten Aspekt der vorliegenden Erfindung umfasst vorzugsweise ein Zimtsäureamid-Derivat der Formel (VII) oder eine Mischung aus mindestens zwei, drei, vier, fünf oder mehreren Zimtsäureamid-Derivaten der Formel (VII).

Gemäß einer bevorzugten Ausgestaltung liegt die Gesamtkonzentration der Zimtsäureamid-Derivate der Formel (VI) in dem Kombinationsmittel bei 100 mg/kg oder weniger, bevorzugt bei 50 mg/kg oder weniger, besonders bevorzugt bei 5 mg/kg oder weniger, jeweils bezogen auf das Gesamtgewicht des Kombinationsmittels. Bei der Bestimmung der Gesamtkonzentration der Zimtsäureamid-Derivate in Mischung ist die Summe der Konzentrationen der einzelnen Verbindungen gemeint.

Die aromatischen Alkensäure-Derivate der Formel (VIII) sind ausgewählt aus der Gruppe, die besteht aus: 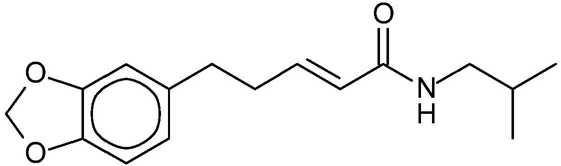 5-(1,3-Benzodioxol-5-yl)-N-isobutyl-pent-2-enamid (Dihydropiperlinguminin, Verbindung 12) 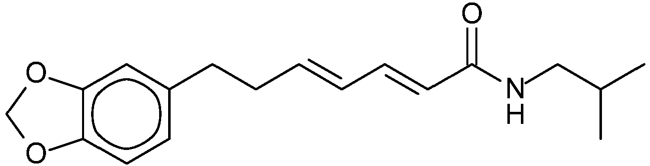 7-(1,3-Benzodioxol-5-yl)-N-isobutyl-hepta-2,4-dienamid (Chingchengenamide A, Verbindung 13): 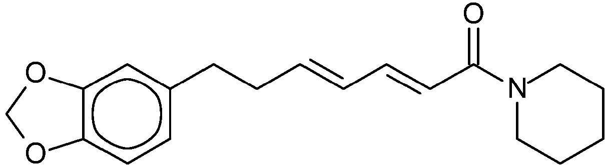 7-(1,3-Benzodioxol-5-yl)-1-(1-piperidyl)hepta-2,4-dien-1-on (Piperdardin, Verbindung 14) und 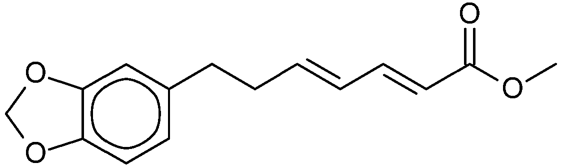 Methyl-7-(1,3-benzodioxol-5-yl)hepta-2,4-dienoat (Verbindung 15).

Die erfindungsgemäßen aromatischen Alkensäure-Derivate der Formel (VIII) liegen vorzugsweise zu mehr als 50 %, besonders bevorzugt zu mehr als 80 %, insbesondere bevorzugt zu mehr als 90 % in der E-Konfiguration vor, wobei die aromatischen Alkensäure-Derivate vorzugsweise ausgewählt sind aus den Verbindungen 12 bis 15.

Das Kombinationsmittel gemäß dem ersten Aspekt der vorliegenden Erfindung umfasst vorzugsweise ein aromatisches Alkensäure-Derivat der Formel (VIII) oder eine Mischung aus mindestens zwei, drei oder mehreren aromatischen Alkensäure-Derivaten der Formel (VIII), welche bevorzugt ausgewählt sind aus der Gruppe der Verbindung 12, Verbindung 13, Verbindung 14 und Verbindung 15. Ebenfalls ist es vorteilhaft, wenn alle vier Verbindungen in einem solchen Kombinationsmittel vorliegen.

Die erfindungsgemäßen aromatischen Alkensäure-Derivate der Formel (VIII) sind in der Lage, bereits in einem Konzentrationsbereich von 0,01 µM bis 10 µM (ca. 0,02 mg/kg bis ca. 2 mg/kg) die Serotonin-Freisetzung in Neuronen um maximal bis zu ca. 300 % gegen die jeweilige Kontrolle anzuregen.

Die jeweiligen Konzentrationen der aromatischen Alkensäure-Derivate sind im Vergleich zu typischen Einsatzkonzentrationen, um einen Geschmackseffekt zu erzielen, gering; es ist also nicht mit stark wahrnehmbaren Aromaeffekten zu rechnen.

Demgemäß ist eine bevorzugte Ausführungsform ein Kombinationsmittel, bei dem die Gesamtkonzentration der aromatischen Alkensäure-Derivate der Formel (VIII) in einem Bereich von 20 mg/kg oder weniger, bevorzugt in einer Konzentration von 5 mg/kg oder weniger, besonders bevorzugt in einer Konzentration von 2 mg/kg oder weniger, vorliegt, bezogen auf das Gesamtgewicht des Kombinationsmittels. Bei der Bestimmung der Gesamtkonzentration der aromatischen Alkensäure-Derivate in der Mischung ist die Summe der Konzentrationen der einzelnen Verbindungen gemeint.

Charakteristisches Merkmal der oben beschriebenen und in dem Kombinationsmittel gemäß der vorliegenden Erfindung erfindungsgemäß verwendeten erste Sättigungskomponente(n) ist, dass sie das Hungergefühl verringern oder gegen Heißhungerattacken wirken, das Sättigungsgefühl steigern und/oder den Stoffwechsel anregen.

Am meisten bevorzugt wird als erste Sättigungskomponente in dem Kombinationsmittel gemäß der vorliegenden Erfindung Nonivamid (N-nonanoylvanillylamin) allein oder in Kombination mit Capsaicin, Zimtaldehyd und/oder trans-Pellitorin verwendet, beispielsweise Nonivamid und Capsaicin und/oder Zimtaldehyd und/oder trans-Pellitorin, oder Capsaicin und Zimtaldeyhd und/oder trans-Pellitorin, oder Zimtaldehyd und trans-Pellitorin. Aufgrund der Serotoninausschüttenden Wirkung, kann mit derartigen Kombinationen als erste Sättigungskomponenten das Hungergefühl besonders stark verringert werden.

Wenn mehrere erste Sättigungskomponenten in Mischung verwendet werden, können die jeweiligen erfindungsgemäß anzuwendenden erste Sättigungskomponenten bei der oben genannten Gesamtkonzentration in dem erfindungsgemäßen Kombinationsmittel jeweils in Einzelkonzentrationen weit, d.h. deutlich unter ihrem jeweiligen sensorischem Erkennungsschwellenwert bei gleichbleibender erfindungsgemäßer Wirkung eingesetzt werden, ohne den Geschmack negativ zu beeinflussen. Da einige der o.g. erste Sättigungskomponenten einen gegenüber Nonivamid sensorisch niedrigeren Erkennungsschwellenwert bei gleichbleibender erfindungsgemäßer Wirkung aufweisen, sind diese in dem erfindungsgemäßen Kombinationsmittel in einer höheren Menge einsetzbar oder können einen Teils des Nonivamids ersetzen, um somit dessen Konzentration unter seinen sensorischen Erkennungsschwellenwert zu senken.

Demgemäß ist eine bevorzugte Ausführungsform ein Kombinationsmittel, bei dem Nonivamid in Kombination mit Capsaicin und/oder Zimtaldehyd und/oder trans-Pellitorin in Einzelkonzentrationen eingesetzt werden, die in der Einsatzkonzentration, d.h. der Gesamtkonzentration in dem erfindungsgemäßen Kombinationsmittel, nur einen unterschwelligen Aromawert zeigen, ohne Verschlechterung der oben beschriebenen physiologischen Effekte.

Unter (Gesamt-)konzentration der erste Sättigungskomponente wird entweder die Konzentration einer erste Sättigungskomponente in dem erfindungsgemäßen Kombinationsmittel verstanden, wenn diese alleine zum Einsatz kommt, oder die Summe der Einzelkonzentrationen der jeweiligen einzelnen erste Sättigungskomponenten, wenn diese in Mischung in dem erfindungsgemäßen Kombinationsmittel zum Einsatz kommen.

Einige der zuvor im Detail beschriebenen erste Sättigungskomponenten, kommen natürlich in bestimmten Pflanzenarten vor, wobei einige der Verbindungen entweder direkt, d.h. als Reinstoff, oder in Form von Extrakten und Zubereitungen gewonnen und verwendet werden. Beispielsweise wurde Verbindung 12 z.B. in *Piper longum* (Stangenpfeffer, Tabuneng W., Bando H., Amiya T., 1983, Chemical & Pharmaceutical Bulletin, 31, 3562 - 3565) gefunden, Verbindung 13 in *P. nigrum* (Friedman M., Levin C. E., Lee S. U., Lee J. S., Ohnisi-Kameyama M., Kozukue N., 2008, J. Agric. Food Chem., 56, 3028 - 3036) und Verbindung 14 in *Piper nigrum* (Schwarzer Pfeffer, Kapoor I. P., Singh B., Singh G., De Heluani C. S., De Lampasona M. P., Catalan C. A., 2009, J. Agric. Food Chem., 57, 5358 - 5364) beschrieben.

Derartige Extrakte können aus den entsprechenden frischen oder getrockneten Pflanzen oder Pflanzenteilen, Blättern, Samen oder Fruchtständen gewonnen werden. Üblicherweise werden hierzu die getrockneten Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0 °C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise enzymatisch behandelt werden, mit Säure (z.B. unter Druck), mit sauren Ionenaustauschern oder mit Wasserdampf behandelt werden, meist bei Drücken von 0,01 mbar bis 100 bar, vorzugsweise bei 1 mbar bis 20 bar, und/oder in einem für Nahrungs- und Genussmittel geeigneten Lösungsmittel aufgenommen werden.

Bevorzugt sind solche Extrakte, in denen die oben beschriebenen erste Sättigungskomponenten den Hauptanteil bezogen auf die Gesamtmenge der extrahierten Verbindungen darstellen, beispielsweise 1 bis 90 Gew.-%, bevorzugt 10 bis 80 Gew.-%, noch mehr bevorzugt 20 bis 70 Gew.-% bezogen auf die Gesamtmenge der extrahierten Verbindungen.

In einer weiteren Variante umfasst das Kombinationsmittel gemäß dem ersten Aspekt der vorliegenden Erfindung daher einen Pflanzenextrakt, z. B. aus *Capsicum-*Arten, der mindestens eine der oben beschriebenen Sättigungskomponente(n) umfasst.

### Bestandteil (b) - zugesetztes freies Arginin oder dessen Isomere oder Derivate

Als weiteren Hauptbestandteil umfasst das Kombinationsmittel gemäß dem ersten Aspekt der vorliegenden Erfindung zugesetztes freies Arginin oder dessen Isomere oder Derivate.

Überraschend wurde festgestellt, dass neben der einen oder den mehreren erste Sättigungskomponente(n) der Zusatz von freiem Arginin in dem Kombinationsmittel gemäß der Erfindung zu einer weiteren synergistisch stärkeren Reduzierung des Appetits und/oder zur Vermittlung eines Sättigungsgefühls führt, verglichen mit einer Zubereitung, die nur eine oder mehrere der oben beschriebenen erste Sättigungskomponente(n) oder nur Arginin umfasst.

Unter zugesetztem freien Arginin wird eine freie, d.h. nicht gebundene, Aminosäure verstanden, die dem Kombinationsmittel zugesetzt wird und nicht selektiv freigesetzt wird aus anderen Protein-Bestandteilen des Kombinationsmittels bzw. nicht aus anderen Protein-Bestandteilen des Kombinationsmittels stammt, insbesondere nicht aus der zweiten Sättigungskomponente des Kombinationsmittels, wie sie weiter unten beschrieben wird.

Die orale Verabreichung von bestimmten freien Aminosäuren kann zu einer Stimulierung der Ausschüttung der Sättigungshormone GLP-1 und PYY- und damit zu einer Abnahme der Energieaufnahme führen. Der vorliegend beschriebene Effekt geht für die Aminosäure Arginin jedoch signifikant über diese Stimulierungseffekte hinaus.

Die Aminosäure Arginin kann entsprechend der möglichen Stereoisomere in der D- und L-Konfiguration vorliegen. Das Kombinationsmittel gemäß der vorliegenden Erfindung schließt somit auch die möglichen Stereoisomere der o.g. Aminosäure sowie Mischungen der Stereoisomere ein.

In einer bevorzugten Ausführungsform kommt in dem erfindungsgemäßen Kombinationsmittel die Aminosäure Arginin in der D- oder L-Konfiguration oder Mischungen daraus zum Einsatz, noch mehr bevorzugt in der enantiomerenreinen L-Konfiguration.

Die Aminosäure Arginin kann auch in Form ihrer Derivate in dem erfindungsgemäßen Kombinationsmittel zur Anwendung kommen, insbesondere in Form ihrer Salze oder Mischungen daraus. Bevorzugt sind die ein- oder zweiwertigen Salze und das Ammoniumsalz. Unter den Salzen sind Natrium-, Calcium-, Magnesium-, Kalium- oder Ammoniumsalze besonders bevorzugt.

Aufgrund ihres Einflusses auf die Protonensekretion, wie es durch das Parietalzellmodell HGT-1 belegt wird, sind überraschender Weise von den in der nachfolgenden Tabelle 1 aufgeführten Aminosäuren L-Arginin und D-Arginin besonders geeignet, den Hunger zu reduzieren und ein nachhaltiges Sättigungsgefühl herbeizuführen. Dieser Effekt wird durch die nachfolgend in Tabelle 1 aufgeführten intrazellulären Protonenindex-Werte (IPX) veranschaulicht.

**Tabelle 1:**

| amino acid | chemical structure | functional group | molecular weight [g/mol] | IPX [induced by 50 mM AA] | | P-value (stereoisomerism) |
|---|---|---|---|---|---|---|
| | | | | L | D | |
| TRP | 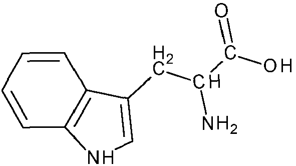 | aromatic | 204.2 | -0.66 ± 0.23 | -0.04 ± 0.04 | < 0.001 |
| PHE* | 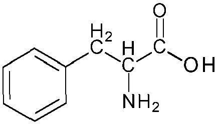 | aromatic | 165.2 | 0.50 ± 0.05 | 1.58 ± 0.10 | < 0.001 |
| ARG* | 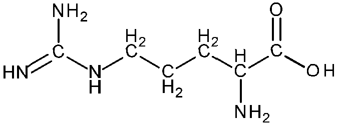 | guanidino | 174.2 | -2.71 ± 0.11 | -2.37 ± 0.05 | < 0.05 |
| ILE* | 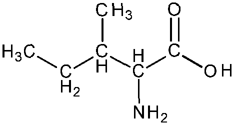 | aliphatic | 131.2 | 0.58 ± 0.07 | -0.40 ± 0.04 | < 0.001 |
| VAL* | 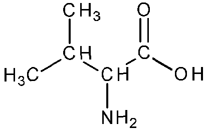 | aliphatic | 117.2 | 0.99 ± 0.05 | -0.44 ± 0.17 | < 0.001 |
| LEU* | 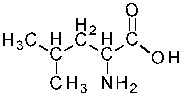 | aliphatic | 131.2 | 1.38 ± 0.14 | 1.10 ± 0.13 | < 0.05 |
| PRO* | 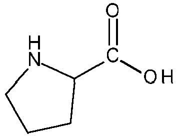 | imino acid | 115.1 | 0.44 ± 0.08 | 0.63 ± 0.09 | p = 0.156 |
| THR* | 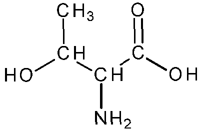 | hydroxyl | 119.1 | 1.43 ± 0.10 | 1.57 ± 0.05 | p = 0.351 |
| SER* | 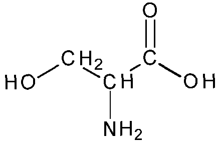 | hydroxyl | 105.1 | 1.40 ± 0.12 | 0.87 ± 0.10 | < 0.01 |
| ALA* | 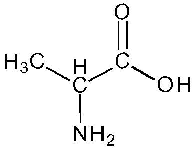 | aliphatic | 89.1 | 0.87 ± 0.10 | -0.35 ± 0.08 | < 0.001 |

Der Wert der intrazellulären Protonensekretion (IPX) im Parietalzellmodell HGT-1 (Protonensekretions-Assay), welches ein Marker für die Magensekretion ist, gibt an, wie stark die Aminosäure die Protonensekretion zu hemmen vermag und ist damit indirekt ein Maß für die Sättigung. Der intrazelluläre Protonenindex (IPX) basiert auf den log2-Daten der intrazellulären Protonenkonzentration. Je niedriger der IPX, desto mehr Protonen werden sezerniert. Ein niedriger IPX wird als Stimulierung der Magensäure gedeutet, welche wiederum ein Sättigungssignal ist.

Von den in Tabelle 1 aufgeführten Aminosäuren weisen L-Arginin und D-Arginin die beste säuremodulierende Wirkung auf, die ein Indikator für eine Sättigung ist.

Die besten Ergebnisse zur Verringerung des Hungergefühls und der raschen Einstellung einer Sättigung werden erzielt, wenn das Kombinationsmittel gemäß dem ersten Aspekt der vorliegenden Erfindung zugesetztes freies L-Arginin oder D-Arginin oder Mischungen daraus umfasst.

Die besten Ergebnisse einer Hunger-reduzierenden und Sättigungs-steigernden Wirkung werden erhalten, wenn das Kombinationsmittel gemäß dem ersten Aspekt der vorliegenden Erfindung zugesetztes freies L-Arginin umfasst.

Besonders vorteilhaft, d.h. wirkungsvoll, ist das Kombinationsmittel, das die zugesetzte freie Aminosäure L-Arginin oder D-Arginin oder Mischungen aus den vorgenannten Aminosäuren in einer Menge von 0,1 bis 20 Gew.-%, bevorzugt von 5 bis 20 Gew.-%, bezogen auf das Gesamtgewicht des Kombinationsmittels, umfasst. Besonders bevorzugt ist eine Menge an zugesetzter freier Aminosäure L-Arginin oder D-Arginin oder Mischungen aus den vorgenannten Aminosäuren von 0,5 bis 10 Gew.-% und am meisten bevorzugt von 2 bis 7 Gew.-%, bezogen auf das Gesamtgewicht des Kombinationsmittels.

Die allerbesten erfindungsgemäßen Effekte werden erzielt mit einem Kombinationsmittel, das die zugesetzte freie Aminosäure L-Arginin in einer Menge von 0,1 bis 20 Gew.-%, bevorzugt von 5 bis 20 Gew.-%, bezogen auf Gesamtgewicht des Kombinationsmittels, umfasst. Besonders bevorzugt ist eine Menge an zugesetzter freier Aminosäure L-Arginin von 0,5 Gew.-% bis 10 Gew.-% und am meisten bevorzugt von 2 bis 7 Gew.-%, bezogen auf das Gesamtgewicht des Kombinationsmittels.

### Optionaler Bestandteil (c) - eine oder mehrere zweite Sättigungskomponente(n)

Besonders vorteilhaft, d.h. wirkungsvoll, ist das Kombinationsmittel gemäß dem ersten Aspekt der vorliegenden Erfindung, insbesondere ein oral konsumierbares Produkt, das optional als weiteren Bestandteil eine oder mehrere zweite Sättigungskomponente(n) umfasst.

Überraschend wurde festgestellt, dass sich bei der Verabreichung des Kombinationsmittels mit einer zweiten Sättigungskomponente der Appetit noch stärker verringern lässt und ein noch schnelleres Sättigungsgefühl auftritt, verglichen mit der Verabreichung desselben Kombinationsmittels ohne zweite Sättigungskomponente.

Wie die erste Sättigungskomponente des erfindungsgemäßen Kombinationsmittels ist die zweite Sättigungskomponente ebenfalls ein zum Verzehr geeigneter Bestandteil mit einer gewichtsreduzierenden Wirkung, wobei die gewichtsreduzierende Wirkung hauptsächlich auf einer Hemmung des Hungerzentrums oder einer Beeinflussung des Sättigungszentrums im Hypothalamus des Gehirns beruht. Ferner kann dieser Bestandteil mit einer sympathomimetischen Wirkung durch Steigerung des Grundumsatzes zur Gewichtsreduktion beitragen.

Bei der zweiten Sättigungskomponente handelt es sich insbesondere um essbare tierische oder pflanzliche Proteine, insbesondere Proteinisolate, oder um essbare tierische oder pflanzliche Proteine in Form von Fraktionen, partiellen oder vollständigen Hydrolysaten oder durch physikalisch-chemische Prozesse oder fermentative oder enzymatische Behandlung der Proteine hergestellte Zwischenprodukte.

In einer bevorzugten Variante umfasst das erfindungsgemäße Kombinationsmittel ein essbares tierisches oder pflanzliches Protein, insbesondere ein Proteinisolat, als die eine oder die mehreren zweite Sättigungskomponente(n). Das essbare tierische oder pflanzliche Protein bzw. Proteinisolat ist ausgewählt aus Proteinen der Gruppe, die besteht aus: Fleisch (Säugetiere, Vögel, Reptilien, Amphibien, Fisch), Krebsen, Schalentieren, Muscheln, Weichtieren, Insekten, Eiern, Milch, insbesondere Casein und Molke, Algen, Getreide, insbesondere Weizen, Gerste, Raps, Sonnenblumen, Reis, Kartoffeln, Mais, Soja, Bohnen, Erbsen, Linsen, Lupinen, Erdnuss, Alfalfa, Hanf und weiteren Proteinen aus essbaren Pflanzen.

Die Proteinisolate können zwischen 30 und 100 % reines Protein, bevorzugt 50 bis 95, besonders bevorzugt 70 bis 90 % reines Protein enthalten und werden durch übliche Methoden gewonnen.

In einer weiteren bevorzugten Variante des Kombinationsmittels ist die eine oder sind die mehreren zweite Sättigungskomponente(n) Proteinhydrolysat(e) ausgewählt aus der Gruppe, die besteht aus Teilhydrolysat oder vollständigem Hydrolysat von Proteinen aus Fleisch (Säugetiere, Vögel, Reptilien, Amphibien, Fisch), Krebsen, Schalentieren, Muscheln, Weichtieren, Insekten, Eiern, Milch, insbesondere Casein und Molke, Algen, Getreide, insbesondere Weizen, Gerste, Raps, Sonnenblumen, Reis, Kartoffeln, Mais, Soja, Bohnen, Erbsen, Linsen, Lupinen, Erdnuss, Alfalfa, Hanf und weiteren Proteinen aus essbaren Pflanzen.

Proteinhydrolysate sind durch chemische oder enzymatische Behandlung gespaltene Proteine. Je nach Größe der Proteinbruchstücke wird zwischen Teilhydrolysat, einem Gemisch aus Peptiden unterschiedlicher Kettenlänge und Aminosäuren, und vollständigem Hydrolysat, einer Mischung aus monomeren Aminosäuren, unterschieden.

Ausgangsprodukte für die Herstellung solcher Proteinisolate, -fraktionen, partiellen oder vollständigen Hydrolysate sind essbare tierische oder pflanzliche Proteine, ausgewählt aus der Gruppe, die besteht aus: Fleisch (Säugetiere, Vögel, Reptilien, Amphibien, Fisch), Krebsen, Schalentieren, Muscheln, Weichtieren, Insekten, Eiern, Milch, insbesondere Casein und Molke, Algen, Getreide, insbesondere Weizen, Gerste, Raps, Sonnenblumen, Reis, Kartoffeln, Mais, Soja, Bohnen, Erbsen, Linsen, Lupinen, Erdnuss, Alfalfa, Hanf und weiteren Proteinen aus essbaren Pflanzen.

Besonders bevorzugt sind Proteinhydrolysate mit einem Hydrolysegrad von mindestens 10 %, bevorzugt mindestens 30 %, besonders bevorzugt größer 50 % der im Proteinanteil gebundenen Aminosäuren.

Als besonders bevorzugte Ausführung werden Proteinhydrolysate gesehen, die durch Peptidase/Hydrolasen hergestellt werden, die ein Präferenz für die Spaltung an N- und/oder C-spezifischen Arginin-Positionen im Protein zeigen und dadurch eine selektive Anreicherung an L-Arginin oder L-Arginin-haltigen kleinen Peptiden erreichen, wie z.B. in Schindler A., Dunkel A., Stähler F., Backes M., Ley J., Meyerhof W., Hofmann T., Discovery of salt taste enhancing arginyl dipeptides in protein digests and fermented fish sauces by means of a sensomics approach, J. Agric. Food Chem., 2011, 59 (23), 12578 - 12588, beschrieben.

Besonders vorteilhafte Effekte der Gewichtsreduktion wurden überraschenderweise festgestellt, wenn in dem Kombinationsmittel gemäß der vorliegenden Erfindung als zweite Sättigungskomponente ein Protein bzw. ein Proteinisolat mit einem intrinsisch hohen L-Arginingehalt oder ein Proteinhydrolysat aus einem Protein mit einem intrinsisch hohen L-Arginingehalt verwendet wird.

Proteine mit intrinsisch hohem L-Arginingehalt sind beispielsweise Proteine aus Kürbiskernen (5353 mg L-Arginin pro 100 g), Walnüssen, Erdnüssen (3460 mg L-Arginin pro 100 g), Mandeln (2750 mg L-Arginin pro 100 g), Pinienkernen (2413 mg L-Arginin pro 100 g), Linsen (2240 mg L-Arginin pro 100 g), Erbsen, Kichererbsen, Haselnüssen (2030 mg L-Arginin pro 100 g), Garnelen (1740 mg L-Arginin pro 100 g), Rindfleisch (1540 mg L-Arginin pro 100 g), Schweinefleisch, Hähnchenbrust (1350 mg L-Arginin pro 100 g), Lachs (1330 mg L-Arginin pro 100 g), Edamer (1030 mg L-Arginin pro 100 g), Buchweizen (970 mg L-Arginin pro 100 g), Mais, Reis, Eier (840 mg L-Arginin pro 100 g) und Kuhmilch (alle vorgenannten Gehaltsangaben beziehen sich auf 100 g des jeweiligen Lebensmittels).

In einer besonders bevorzugten Ausführungsform gemäß der vorliegenden Erfindung umfasst das Kombinationsmittel daher als zweite Sättigungskomponente ein Protein bzw. Proteinisolat mit einem hohen intrinsischen L-Arginingehalt oder ein Proteinhydrolysat aus einem Protein mit einem hohen intrinsischen L-Arginingehalt.

Am meisten bevorzugt ist ein Protein bzw. Proteinisolat oder ein Proteinhydrolysat als zweite Sättigungskomponente, das einen intrinsischen L-Arginingehalt von mindestens 800 mg L-Arginin pro 100 g aufweist, noch mehr bevorzugt von mindestens 1000 mg L-Arginin pro 100 g und am meisten bevorzugt von mindestens 2500 mg L-Arginin pro 100 g Lebensmittel umfasst.

Die Menge der einen oder mehreren zweite Sättigungskomponente in dem Kombinationsmittel, das die Bestandteile (a), (b) und (c) umfasst, beträgt 1 bis 95 Gew.-%, vorzugsweise 1 bis 80 Gew.-% und besonders bevorzugt 5 bis 75 Gew.-%, bezogen auf das Gesamtgewicht des Kombinationsmittels.

Ein niedriger intrinsischer L-Arginingehalt des als zweite Sättigungskomponente verwendeten Proteins bzw. Proteinisolats oder des Proteinhydrolysats kann durch den Zusatz von freiem L-Arginin kompensiert oder vorteilhafter Weise erhöht werden. Dies geschieht zum einen durch den Zusatz von freiem L-Arginin über den Bestandteil (b) des Kombinationsmittels oder durch eine Anreicherung des Proteins bzw. Proteinisolats oder Proteinhydrolysats mit freiem L-Arginin über den Bestandteil (c).

In der zuletzt genannten vorteilhaften Variante des Kombinationsmittels gemäß dem ersten Aspekt der vorliegenden Erfindung ist daher in dem Kombinationsmittel der Gesamtgehalt an L-Arginin höher als der natürliche intrinsische L-Arginingehalt des Proteinbestandteils (Protein bzw. Proteinisolat oder Proteinhydrolysat) der zweiten Sättigungskomponente. Der Gesamtgehalt an L-Arginin in dem Kombinationsmittel ist somit eine Summe aus dem Gehalt an zugesetztem freien L-Arginin (Bestandteil (b)) plus dem natürlichen intrinsischen L-Arginingehalt des Proteinbestandteils der zweiten Sättigungskomponente (Bestandteil (c)).

Die Menge an zugesetztem freien L-Arginin in dem Kombinationsmittel, das die Bestandteile (a), (b) und (c) umfasst, beträgt vorzugsweise 0,1 bis 20 Gew.-%, noch mehr bevorzugt 5 bis 20 Gew.-%, bezogen auf das Gesamtgewicht des Kombinationsmittels. Besonders bevorzugt ist eine Menge an zugesetztem freien L-Arginin von 0,5 bis 10 Gew.-% und am meisten bevorzugt 2 bis 7 Gew.-%, bezogen auf das Gesamtgewicht des Kombinationsmittels, um eine Hunger-reduzierende und Sättigungs-steigernde Wirkung zu erreichen.

Damit der erfindungsgemäße Effekt eintritt, beträgt vorteilhafter Weise der Gesamtgehalt an L-Arginin in dem erfindungsgemäßen Kombinationsmittel in einer regulär konsumierbaren Menge (Portionsgröße), das die Bestandteile (a) und (b) oder die Bestandteile (a), (b) und (c) umfasst, mindestens 0,1 g L-Arginin, noch mehr bevorzugt mindestens 0,5 g L-Arginin und am meisten bevorzugt mindestens 1 g L-Arginin, bezogen auf das Gesamtgewicht der regulär konsumierbaren Menge (Portionsgröße) des Kombinationsmittels.

### Optionaler Bestandteil (d) - eine oder mehrere sensorisch aktive Substanz(en)

In einer weiteren Variante gemäß dem ersten Aspekt der vorliegenden Erfindung umfasst das erfindungsgemäße Kombinationsmittel optional eine oder mehrere sensorisch aktive Substanz(en), die ausgewählt ist/sind aus der Gruppe, die besteht aus süßenden Lebensmitteln, Süßstoffen, Aromastoffen und Geschmacksmodulatoren. Mit dem Zusatz einer oder mehreren der vorgenannten Substanz(en) lassen sich das Geschmacks- bzw. das Aromaprofil des erfindungsgemäßen Kombinationsmittels modifizieren oder ausbalancieren.

Mit der Verwendung von einem oder mehreren süßenden Lebensmittel(n) als sensorisch aktive Substanz kann in dem erfindungsgemäßen Kombinationsmittel ein Süßeindruck erzeugt werden. Die in dem erfindungsgemäßen Kombinationsmittel verwendeten süßenden Lebensmittel sind vorzugsweise ausgewählt aus der Gruppe, die besteht aus: flüssige oder trockene Konzentrate, bevorzugt Pulver, von Süßmolke, Süßkartoffel, Honig, Agave, Akazie, Fruchtdicksäften, insbesondere aus Banane, Weintraube, Apfel, Aprikose, Dattel, Zuckerrübensirup und Milchzucker.

Mit dem Zusatz von Süßstoffen lässt sich das erfindungsgemäße Kombinationsmittel beispielweise süßen, ohne dabei den Energiegehalt des Kombinationsmittels zu erhöhen. Darüber hinaus lässt sich mit dem Zusatz beispielsweise ein unangenehmer, insbesondere ein bitterer Geschmack der übrigen Bestandteile, beispielsweise der ersten Sättigungskomponente oder der freien Aminosäure, des Kombinationsmittels, verringern oder unterdrücken.

Der Begriff "Süßstoffe" bezeichnet solche Stoffe mit einer relativen Süßkraft von zumindest 25, bezogen auf die Süßkraft von Saccharose (welches somit die Süßkraft 1 hat). Vorzugsweise sind in der erfindungsgemäßen Zusammensetzung einzusetzende Süßstoffe nicht-kariogen und/oder besitzen einen Energiegehalt von maximal 5 kcal pro Gramm der erfindungsgemäßen Zusammensetzung.

Vorteilhafte Süßstoffe in einer bevorzugten erfindungsgemäßen Zusammensetzung sind ausgewählt aus den folgenden Gruppen (a1) und (a2):
(a1) natürlich vorkommende Süßstoffe, vorzugsweise ausgewählt aus der Gruppe bestehend aus
(a1-1) Miraculin, Monellin, Mabinlin, Thaumatin, Curculin, Brazzein, Pentaidin, D-Phenylalanin, D-Tryptophan, Glycin, D-Threonin, D-Serin und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Rabaudiosid M, Rebaudiosid N, Rebaudiosid O, Rebaudiosid P, Rebaudiosid R, Rebaudiosid X, Rebaudiosid Z, Dulcosiden und Rubusosid, Suaviosid A, Suaviosid B, Suaviosid G, Suaviosid H, Suaviosid I, Suaviosid J, Baiyunosid 1, Baiyunosid 2, Phlomisosid 1, Phlomisosid 2, Phlomisosid 3 und Phlomisosid 4, Abrusosid A, Abrusosid B, Abrusosid C, Abrusosid D, Cyclocaryosid A und Cyclocaryosid I, Osladin, Polypodosid A, Strogin 1, Strogin 2, Strogin 4, Selligueain A, Dihydroquercetin-3-acetat, Perillartin, Telosmosid A₁₅, Periandrin I-V, Pterocaryosiden, Cyclocaryosiden, Mukuroziosiden, trans-Anethol, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivate, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a1-3) Extrakten oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana),* Swingle-Extrakten *(Momordica* oder *Siratia grosvenorii,* Luo-Han-Guo), Extrakten aus *Glycerrhyzia* ssp. (insbesondere *Glycerrhyzia glabra),* Extrakten aus *Rubus* ssp. (insbesondere *Rubus suavissimus),* Citrus-Extrakten und Extrakten aus *Lippia dulcis;* und
(a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfams, Neohesperidindihydrochalkon, Naringindihydrochalkon, Saccharin, Saccharin-Natriumsalz, Aspartam, Superaspartam, Neotam, Alitam, Advantam, Perillartin, Monellin, Sucralose, Lugdunam, Carrelam, Sucrononat und Sucrooctat.

In einer weiteren Ausgestaltung der vorliegenden Erfindung wird in dem erfindungsgemäßen Kombinationsmittel optional wenigstens ein Aromastoff als sensorisch aktive Substanz, insbesondere ein Aroma, verwendet, um das erfindungsgemäße Kombinationsmittel zu aromatisieren. Das Aroma kann dabei fest oder flüssig sein.

In einer bevorzugten Ausführungsform umfasst das erfindungsgemäße Kombinationsmittel ein Aroma, um dessen Geschmack und/oder Geruch der Zusammensetzung abzurunden und/oder zu verfeinern. Geeignete Aromen werden bevorzugt ausgewählt aus der Gruppe bestehend aus synthetischen, natürlichen oder naturidentischen Aroma-, Riech- und Geschmacksstoffen und deren Gemische, ätherischen Ölen, Reaktionsaromen, Raucharomen oder andere aromagebende Zubereitungen. Dabei kann es sich weiterhin vorzugsweise um Protein[teil]hydrolysate, Grillaromen, Pflanzenextrakte, Gewürze, Gewürzzubereitungen, Gemüse und/oder Gemüsezubereitungen handeln, die ferner geeignete Hilfs- und Trägerstoffe enthalten können. Insbesondere sind erfindungsgemäß bevorzugt Aromen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit einen würzigen und salzigen Eindruck verstärken können.

Ätherische Öle, die erfindungsgemäß Bestandteil des Aromas sein können, werden vorzugsweise gewählt aus der Gruppe bestehend aus Anisöl; Baldrianöl; Basilikumöl; Beifußöl; Bergamotteöl; Bittermandelöl; Bohnenkrautöl; Buccoblätteröl; Campheröl; Canangaöl; Cardamomenöl; Cascarillaöl; Cassiaöl; Citronellöl; Citronenöl; Corianderöl; Cuminöl; Dillkrautöl; Dillsamenöl; Estragonöl; Eucalyptus-citriodora-Öl; Eucalyptusöl; Fenchelöl; Grapefruitöl; Ingweröl; Kamillenöl blau; Kamillenöl römisch; Krauseminzöl; Kümmelöl; Lemongrasöl; Liebstocköl; Limettenöl destilliert; Limettenöl gepresst; Lorbeerblätteröl; Macisöl; Majoranöl; Mandarinenöl; Muskatnußöl; Nelkenblätteröl; Nelkenblütenöl; Orangenöl; Origanumöl; Petersilienblätteröl; Petersiliensamenöl; Pfefferminzöl; Pfefferöl; Pimentöl; Rosmarinöl; Salbeiöl dalmatinisch; Salbeiöl spanisch; Selleriesamenöl; Sternanisöl; Thymianöl; Vanilleextrakt; Wacholderbeeröl; Wintergrünöl; Zimtblätteröl; Zimtrindenöl sowie Fraktionen davon.

Im Rahmen der vorliegenden Erfindung werden vorzugsweise auch Reaktionsaromen verwendet, die Bestandteil des Aromas sein können, die durch Bratung eines Gewürzes oder eines Tresters hergestellt werden können, sind vorzugsweise ausgewählt aus der Gruppe, die besteht aus Aromen aus Bratung von pflanzlichen Ölen mit Kartoffeln, Süßkartoffeln, Karottentrestern, Zwiebeltrestern, Zuckerrübentrestern, Gemüsetrestern, Fruchttrester oder Pilztrestern.

Im Rahmen der vorliegenden Erfindung werden bevorzugt einzusetzende Einzel-Aromastoffe ausgewählt aus der Gruppe bestehend aus: Acetaldehyd, Acethylmethylcarbinol, Acetophenon, Allylcapronat, alpha Ionon, beta Ionon, Anisaldehyd, Anisylacetat, Anisylformiat, Benzaldehyd, Benzothiazol, Benzylacetat, Benzylacetat, Benzylalkohol, Benzylbenzoat, beta Ionon, Butylbutyrat, Butylcapronat, Butylidenphthalid, Caprinsäure, Capronsäure, Caprylsäure, Carvon, Camphen Caryophyllen, Cineol, Cinnamylacetat, Citral, Citronellol, Citronellal, Citronellylacetat, Cyclohexylacetat, Cymol, Damascon, Decalacton, Diacetyl, Dihydrocumarin, Dimethylanthranilat, Dimethylanthranilat, Dodecalacton, Ethoxyethylacetat, Ethylacetat, Ethylbuttersäure, Ethylbutyrat, Ethylcaprinat, Ethylcapronat, Ethylcrotonat, Ethylformiat, Ethylfuraneol, Ethylguajakol, Ethylisobutyrat, Ethylisovalerianat, Ethyllactat, Ethyllactat, Ethylmaltol, Ethylmethylbutyrat, Ethylpropionat, Eucalyptol, Eugenol, Ethylheptylat, 1-(4'-Hydroxyphenyl)-2-butanon (Frambinor<^{®}>), gamma-Decalacton, Geraniol, Geranylacetat, Geranylacetat, Grapefruitaldehyd, Methyldihydrojasmonat (Hedion<^{®}>), Heliotropin, 2-Heptanon, 3-Heptanon, 4-Heptanon, trans-2- Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cic-3-Hexenylcapronat, trans-2-Hexenylcapronat,cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Maltol, I-Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans,2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (3-Ethoxy-4-isobutyryloxybenzaldehyd), Furaneol<^{®}>(2,5-Dimethyl-4-hydroxy-3(2H)-furanon) und dessen Abkömmlinge (dabei vorzugsweise Homofuraneol ( 2-Ethyl-4-hydroxy-5-methyl-3(2H)-furanon), Homofuronol ( 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalalcton), delta-Lactone (vorzugsweise 4-Methyldeltadecalacton, Massoilacton, Deltadecalacton, Tuberolacton), Methylsorbat, Divanillin, 4-Hydroxy-2(oder 5)-ethyl-5(oder2)-methyl-3(2H)-furanon, 2-Hydroxy-3-methyl-2-cyclopentenon, 3-Hydroxy-4,5-dimethyl-2(5H)-furanon, Essigsäureisoamylester, Buttersäureethylester, Buttersäuren-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)-furan, 2-Methyl-3-furanthiol, Bis(2-methyl-3-furyl)-disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, Mercapto-3-methyl-1-butanol 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 2,3-Butandion, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Dimethylsulfid, Trimethylamin, Zimtaldehyd, Zimtalkohol, Methylsalicylat, Isopulegol sowie - soweit möglich - deren cis/trans-Isomere, Stereoisomere, Enantiomere, Diastereomere und Epimere.

Darüber hinaus können die erfindungsgemäßen verwendeten Aromen solche sein, die vorzugsweise ausgewählt sind aus der Gruppe, die besteht aus Ei, Butter, Röstnoten, Karamell und Popcorn.

Bevorzugt enthalten die erfindungsgemäß einzusetzenden Aromen mehr als einen der genannten Aromastoffe, vorzugsweise zwei, drei, vier, fünf oder mehr der genannten Aromastoffe.

In einer weiteren Ausgestaltung der vorliegenden Erfindung wird in dem erfindungsgemäßen Kombinationsmittel optional wenigstens ein Geschmacksmodulator verwendet. Geschmacksmodulatoren sind solche Substanzen, die in der Lage sind, einen unangenehmen (bitteren, metallischen, kalkigen, sauren, adstringierenden, scharfen) Geschmackseindruck zu maskieren oder zu vermindern, einen angenehmen Geschmackseindrucks (süß, salzig, Umami oder kribbelnder "tingling" oder kühlender Effekt) zu verstärken oder zu erzeugen oder einen Geschmackseindruck zu korrigieren. Damit lässt sich eine Modulation des Geschmacks der erfindungsgemäßen Zusammensetzung erreichen.

Diese weiteren modulierenden Aroma- und/oder Geschmacksstoffe (Geschmacksmodulatoren) als sensorisch aktive Substanz können aus den folgenden Listen ausgewählt werden:

Trans-tert-Butylcyclohexanol (wie beschrieben in WO 2009/087242, die bezüglich der darin offenbarten entsprechenden Verbindungen auf dem Wege der Verweisung Bestandteil dieser Anmeldung wird); Mononatriumglutamat, Glutaminsäure; Nucleotide, wie zum Beispiel Adenosin-5'-monophosphat, Cytidin-5'-monophosphat, Inosin-5'-monophosphat, Guanosin-5'-monophosphat und deren pharmazeutisch akzeptable Salze; Lactisole; Natriumsalze, wie zum Beispiel Natriumchlorid, Natriumlactat, Natriumcitrat, Natriumacetat, Natriumgluconat; Hydroxyflavanone, wie zum Beispiel Eriodictyol, Sterubin (Eriodictyol-7-methylether), Homoeriodictyol und deren Natrium-, Kalium-, Calcium-, Magnesium- oder Zinksalze (insbesondere solche, wie beschrieben in EP 1 258 200, die bezüglich der darin offenbarten entsprechenden Verbindungen auf dem Wege der Verweisung Bestandteil dieser Anmeldung wird); Hydroxybenzoesäureamide, wie zum Beispiel 2,4-Dihydroxybenzoesäurevanillylamid, 2,4-Dihydroxybenzoesäure-N-(4-hydroxy-3-methoxybenzyl)-amid, 2,4,6-Trihydroxybenzoesäure-N-(4-hydroxy-3-methoxybenzyl)-amid, 2-Hydroxybenzoesäure-N-4-(hydroxy-3-methoxybenzyl)-amid, 4-Hydroxybenzoesäure-N-(4-hydroxy-3-methoxybenzyl)-amid, 2,4-Dihydroxybenzoesäure-N-(4-hydroxy-3-methoxybenzyl)-amid-mono-natriumsalz, 2,4-Dihydroxybenzoesäure-N-2-(4-hydroxy-3-methoxyphenyl)-ethylam id, 2,4-Dihydroxybenzoesäure-N-(4-hydroxy-3-ethoxybenzyl)-amid, 2,4-Dihydroxybenzoesäure-N-(3,4-dihydroxybenzyl)-amid und 2-Hydroxy-5-methoxy-N-[2-(4-hydroxy-3-methoxyphenyl)-ethyl]-amid); 4-Hydroxybenzoesäurevanillylamid (insbesondere solche, wie beschrieben in WO 2006/024587, die bezüglich der darin offenbarten entsprechenden Verbindungen auf dem Wege der Verweisung Bestandteil dieser Anmeldung wird); Hydroxydeoxybenzoine, wie zum Beispiel 2-(4-Hydroxy-3-methoxyphenyl)-1-(2,4,6-trihydroxyphenyl)-ethanon, 1-(2,4-Dihydroxyphenyl)-2-(4-hydroxy-3-methoxy-phenyl)-ethanon, 1-(2-Hydroxy-4-methoxyphenyl)-2-(4-hydroxy-3-methoxyphenyl)-ethanon) (insbesondere solche, wie beschrieben in WO 2006/106023, die bezüglich der darin offenbarten entsprechenden Verbindungen auf dem Wege der Verweisung Bestandteil dieser Anmeldung wird); Hydroxyphenylalkandione, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche, wie beschrieben in WO 2007/003527, die bezüglich der darin offenbarten entsprechenden Verbindungen auf dem Wege der Verweisung Bestandteil dieser Anmeldung wird); Diacetyltrimere (insbesondere solche, wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche, wie beschrieben in WO 2005/096841, die bezüglich der darin offenbarten entsprechenden Verbindungen auf dem Wege der Verweisung Bestandteil dieser Anmeldung wird); Divanilline (insbesondere solche, wie beschrieben in WO 2004/078302, die bezüglich der darin offenbarten entsprechenden Verbindungen auf dem Wege der Verweisung Bestandteil dieser Anmeldung wird); 4-Hydroxydihydrochalkone (insbesondere solche, wie beschrieben in US 2008 0227867, die bezüglich der darin offenbarten entsprechenden Verbindungen auf dem Wege der Verweisung Bestandteil dieser Anmeldung wird); in diesem Zusammenhang insbesondere Phloretin und Davidigenin; Aminosäuren oder Mischungen von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen sowie Mischungen (insbesondere solche, wie beschrieben in WO 2007/045566, die bezüglich der darin offenbarten Verbindungen auf dem Wege der Verweisung Bestandteil dieser Anmeldung wird); Hesperetin (insbesondere solche, wie offenbart in WO 2007/014879, die bezüglich der darin offenbarten entsprechenden Verbindungen auf dem Wege der Verweisung Bestandteil dieser Anmeldung wird); 4-Hydrochalkone (insbesondere solche, wie offenbart in WO 2007/107596, die bezüglich der darin offenbarten entsprechenden Verbindungen auf dem Wege der Verweisung Bestandteil dieser Anmeldung wird); Propylenphenylglycoside (Chavicolglycoside) (insbesondere solche, wie beschrieben in EP 1 955 601, die bezüglich der darin offenbarten entsprechenden Verbindungen auf dem Wege der Verweisung Bestandteil dieser Anmeldung wird); Pellitorin und davon abgeleitete Aromazusammensetzungen; Umami-Verbindungen (insbesondere solche, wie beschrieben in WO 2008/046895 und EP 1 989 944, die bezüglich der darin offenbarten Verbindungen auf dem Wege der Verweisung Bestandteil dieser Anmeldung werden); Matairesinol und Neoflavanoide (insbesondere solche, wie beschrieben in WO 2012/146584, US 2013 078192 und US 2013 084252, die bezüglich der darin offenbarten Verbindungen auf dem Wege der Verweisung Bestandteil dieser Anmeldung werden); und Neoisoflavonoide (insbesondere solche, wie beschrieben in EP 2 570 035, EP 2 570 036 und EP 2 726 026, die bezüglich der darin offenbarten Verbindungen auf dem Wege der Verweisung Bestandteil dieser Anmeldung werden), Extrakte aus Mycetia ssp. oder Balansine, wie in EP 2 529 633 B1 beschrieben, Hesperetindihydrochalkon wie in WO 2017 186,299 beschrieben, Mischungen, enthaltend Naringenin und Phloretin wie in WO 2017 092,796 bechriebn, native oder transglycosidierte Rubusoside wie in EP 2 386 211 B1 oder WO 2015 189,346, Flavane wie in US 2010 292,175 beschrieben, Phyllodulcin oder Hydrangea-Extrakte wie in EP 2,298,084 beschrieben, Dihydrochaklkone wie in EP 2,353,403 beschrieben, Homovanillinsäureester wie in EP2,932,858 beschrieben, Phyllodulcinabbauprodukte wie in PCT/EP2017/065457 beschrieben.

Bicyclo [4.1.0]heptan-7-carbonsäureamide, insbesondere solche, wie beschrieben in EP 2 079 322 (Symrise), die bezüglich der darin offenbarten entsprechenden Verbindungen auf dem Wege der Verweisung Bestandteil dieser Anmeldung wird; Cyclopropancarbonsäure(3-methylcyclohexyl)-amide, insbesondere solche, wie beschrieben in EP 1 989 944 (Symrise), die bezüglich der darin offenbarten entsprechenden Verbindungen auf dem Wege der Verweisung Bestandteil dieser Anmeldung wird; aromatische Neo-Menthylamide, insbesondere solche, wie beschrieben in EP 2 064 959 (Symrise), die bezüglich der darin offenbarten entsprechenden Verbindungen auf dem Wege der Verweisung Bestandteil dieser Anmeldung wird; Neo-Menthylamide, insbesondere solche, wie beschrieben in US 2009 311401 (Symrise), die bezüglich der darin offenbarten entsprechenden Verbindungen auf dem Wege der Verweisung Bestandteil dieser Anmeldung wird; Geranylaminderivate der Oxalsäure sowie Neomenthylderivate.

Generell ist der bzw. sind die zwei, drei, mehreren oder sämtliche der weiteren Geschmacksstoffe zum Vermitteln, Modifizieren und/oder Verstärken einer, zwei oder sämtlicher der Geschmackseindrücke Umami, Kokumi und salzig (Geschmacksmodulatoren) vorzugsweise natürlich vorkommende Verbindungen, besonders bevorzugt Verbindungen ausgewählt aus der Gruppe bestehend aus: Mononatriumglutamat, freie Glutaminsäure, Nucleotide, wie zum Beispiels Adenosin-5'-monophosphat, Cytidin-5'-monophosphat, Inosin-5'-monophosphat, Guanosin-5'-monophosphat) und deren pharmazeutisch akzeptable Salze; Strombine (insbesondere solche, wie beschrieben in WO 2010/100589, die bezüglich der darin offenbarten entsprechenden Verbindungen auf dem Wege der Verweisung Bestandteil dieser Anmeldung wird); Theogalline (insbesondere solche, wie beschrieben in JP 2007 110988, die bezüglich der darin offenbarten entsprechenden Verbindungen auf dem Wege der Verweisung Bestandteil dieser Anmeldung wird); Pyridin-Betain-Verbindungen (insbesondere solche, wie beschreiben in EP 1 291 342, die bezüglich der darin offenbarten entsprechenden Verbindungen auf dem Wege der Verweisung Bestandteil dieser Anmeldung wird); Glutaminsäureglycoside (insbesondere solche, wie beschrieben in WO 2002/087361, die bezüglich der darin offenbarten entsprechenden Verbindungen auf dem Wege der Verweisung Bestandteil dieser Anmeldung wird); Äpfelsäureglycoside (insbesondere solche, wie beschrieben in WO 2006/003107, die bezüglich der darin offenbarten entsprechenden Verbindungen auf dem Wege der Verweisung Bestandteil dieser Anmeldung wird); Glutathion-Derivate (insbesondere solche, wie beschrieben in EP 0 181 421 oder WO 2007/042273, die bezüglich der darin offenbarten entsprechenden Verbindungen auf dem Wege der Verweisung Bestandteil dieser Anmeldung werden); Lactisole und Alkylpyridine (insbesondere solche Alkylpyridine, wie beschrieben in WO 2009/122318 und WO 2009/1223319, die bezüglich der darin offenbarten entsprechenden Verbindungen auf dem Wege der Verweisung Bestandteil dieser Anmeldung werden), insbesondere 2-Hexyl-, 2-Heptyl- und 2-Octylpyridin, (2E,6Z)-N-cyclopropylnona-2,6-dienamid, (2E,6Z)-N-ethylnona-2,6-dienamid, N-[(2E)-3,7-dimethylocta-2,6-dienyl]-cyclopropancarboxamid, N'-[(2-methoxy-4-methyl-phenyl)methyl]-N-[2-(5-methyl-2-pyridyl)ethyl]-oxamid, N'-[(2,4-dimethoxyphenyl)-methyl]-N-[2-(2-pyridyl)-ethyl]-oxamid, N'-[(2-methoxy-4-methyl-phenyl)-methyl]-N-[2-(2-pyridyl)-ethyl]-oxamid, N-(1-propylbutyl)-1,3-benzodioxol-5-carboxamid, 1-(2-hydroxy-4-isobutoxy-phenyl)-3-(2-pyridyl)-propan-1-on und 1-(2-hydroxy-4-methoxy-phenyl)-3-(2-pyridyl)-propan-1-on; Zimtsäureamide, insbesondere Rubemamid oder Rubescenamid; Hydroxyflavone, wie zum Beispiel Eriodictyol, Homeriodictyol oder deren Natriumsalze); Hesperetin, Pholoeretin, Hydroxyflavane, 4-Hydroxychalkone; Extrakte auf Basis von Hydrangea dulcis (insbesondere solche, wie beschrieben in EP 2 298 084, die diesbezüglich auf dem Wege der Verweisung Bestandteil dieser Anmeldung wird) oder Rubus ssp.; Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen (insbesondere Mineralsalzmischungen, wie beschrieben in US 2009 214728, die diesbezüglich auf dem Wege der Verweisung Bestandteil dieser Anmeldung wird).

Synthetische vorzugsweise in dem erfindungsgemäßen Kombinationsmittel zu verwendende Geschmacksstoffe zum Vermitteln, Modifizieren und/oder Verstärken einer, zwei oder sämtlicher der Geschmackseindrücke Umami, Kokumi und salzig sind vorzugsweise ausgewählt aus den chemischen Substanzen, die in den Veröffentlichungen US 2004 0202619, US 2004 0202760, US 2006 0057268 und US 2006 0068071 beschrieben sind, insbesondere (2E, 6Z)-N-cyclopropylnona-2,6-dienamid, (2E,6Z)-N-ethylnona-2,6-dienamid und N-[(2E)-3,7-dimethylocta-2,6-dienyl]-cyclopropancarboxamid, den chemischen Strukturen wie in US 2005 0084506 beschrieben, insbesondere N'-[(2-methoxy-4-methylphenyl)-methyl]-N-[2-(5-methyl-2-pyridyl)ethyl]-oxamid, N'-[(2,4-dimethoxyphenyl)-methyl]-N-[2-(2-pyridyl)-ethyl]-oxamid, N'-[(2-methoxy-4-methylphenyl)-methyl]-N-[2-(2-pyridyl)ethyl]-oxamid, N-(1-propylbutyl)-1,3-benzodioxole-5-carboxamid, und den chemischen Strukturen wie in WO 2011/004016 beschrieben, insbesondere 1-(2-Hydroxy-4-isobutoxyphenyl)-3-(2-pyridyl)-propan-1-on und 1-(2-Hydroxy-4-methoxyphenyl)-3-(2-pyridyl)-propan-1-on; Rubemamin, Rubemamid, Rebuscenamin, Rubescenamid, Zanthosin, Zanthosinamid, Dioxamin, Dioxamid, Zanthomamin und Zanthomamid sowie deren Gemische wie in EP 2 529 632 oder EP 2 737 807 beschrieben.

Zusätzlich zu einem oder mehreren süßverstärkenden Stoff(en) oder zu einem oder mehreren Geschmacksmodulator(en) können in dem erfindungsgemäßen Kombinationsmittel vorzugsweise Geschmacksstoffe enthalten sein, die einen kribbelnden (tingling) oder kühlenden Effekt bewirken. Solche Geschmacksstoffe sind vorzugsweise gewählt aus der Gruppe, die besteht aus: Menthol und Mentholderivative wie zum Beispiel L-Menthol, D-Menthol, racemisches Menthol, Isomenthol, Neoisomenthol, Neomenthol; Menthylether wie zum Beispiel 3-(I-Menthoxy)-1,2-propandiol, 3-(I-Menthoxy)-2-methyl-1,2-propandiol, 3-(I-Menthylmethylether; Menthonglycerylacetal, Menthonglycerylketal oder Mischungen davon; Menthylester wie zum Beispiel Menthylformiat, Menthylacetat, Menthylisobutyrat, Menthyhydroxyisobutyrat, Menthyllactate, L-menthyl-L-lactat, L-Menthyl-D-lactat, Menthyl-(2-methoxy)acetat, Menthyl-(2-methoxyethoxy)-acetat, Menthylpyroglutamat; Menthylcarbonate wie zum Beispiel Menthylpropyleneglycolcarbonat, Menthylethyleneglycolcarbonat, Menthylglycerolcarbonat oder Mischungen davon; die Halbester von Mentholen mit einer Dicarbonsäure oder Derivate davon wie zum Beispiel Monomenthylsuccinat, Monomenthylglutarat, Monomenthylmalonat, O-Menthyl-bernsteinsäureester-N,N-(dimethyl)amid, O-Menthyl-bernsteinsäureesteramid), Menthancarbonsäuremaide (vorzugsweise Menthancarbonsäure-N-ethylamid or N^{α}-(Menthancarbonsäure) glycinethylester, wie beschrieben in US 4 150 052, Menthancarbonsäure-N-(4-cyanophenyl)-amid oder Menthancarbonsäure -N-(4-cyanomethylphenyl)-amid, wie beschrieben in WO 2005/049553); Menthancarbonsäure-N-(alkoxyalkyl)-amide); Menthon und Menthonederivative wie zum Beispiel L-Menthoneglycerinketal); 2,3-Dimethyl-2-(2-propyl)-buttersäurederivate wie zum Beispiel 2,3-Dimethyl-2-(2-propyl)-buttersäure-N-methylamid, Isopulegol oder seine Ester wie beispielsweise (I-(-)-Isopulegol, I-(-)-isopulegolacetat; Menthanderivative wie zum Beispiel p-Menthan-3,8-diol; Cubebol oder synthetische oder natürliche Mischungen, die Cubebol enthalten; Pyrrolidonderivative von Cycloalkyldionderivativen wie zum Beispiel 3-Methyl-2(1-pyrrolidinyl)-2-cyclopentene-1-on oder Tetrahydropyrimidine-2-one wie zum Beispiel Icilin or verwandten Verbindungen, wie beschrieben in WO 2004/026840); Carboxamide wie zum Beispiel N-(2-(pyridin-2-yl)-ethyl)-3-p-menthanecarboxamid oder verwandten Verbindungen wie zum Beispiel (1R,2S,5R)-N-(4-Methoxyphenyl)-5-methyl-2-(1-isopropyl)cyclohexancarboxamide; Oxamate, vorzugsweise solche beschrieben in EP 2 033 688 und [(1R,2S,5R)-2-isopropyl-5-methyl-cyclohexyl] 2-(ethylamino)-2-oxo-acetate (X Cool).

### Optionaler Bestandteil (e) - Trägerstoff und/oder Hilfsstoff bzw. Adjuvans:

In einer weiteren Variante kann das Kombinationsmittel gemäß dem ersten Aspekt der vorliegenden Erfindung als weiteren Bestandteil wenigstens einen zum Verzehr geeigneten Trägerstoff und/oder pharmazeutisch wirksamen Hilfsstoff bzw. Adjuvans umfassen.

Der erfindungsgemäß in dem Kombinationsmittel einzusetzende wenigstens eine Trägerstoff wird bevorzugt gewählt aus der Gruppe, die besteht aus: Siliciumdioxid (Kieselsäure, Kieselgel); Kohlenhydraten und/oder Kohlenhydratpolymeren (Polysacchariden); insbesondere unter physiologischen Bedingungen unlöslichen Ballaststoffen, insbesondere ausgewählt aus der Gruppe, die besteht aus: Cellulose, modifizierte Cellulose, Hemicellulosen, Lichenin, Chitin, Chitosan, Ligninen, Xanthan, pflanzliche Fasern, insbesondere Getreidefasern, Kartoffelfasern, Apfelfasern, Citrusfasern, Bambusfasern, extrahierte Zuckerrübenfasern, Haferfasern; unter physiologischen Bedingungen löslichen Ballaststoffen, insbesondere ausgewählt aus der Gruppe, die besteht aus: Inulin, insbesondere nativem Inulin, hochlöslichem Inulin, granuliertem Inulin, Hochleistungs-(high performance) Inulin, Pektine, Alginate, Agar, Carrageen, Gummi arabicum, Guarkernmehl, Johannisbrotkernmehl, Xanthan, Raffinose, Xylose, Polydextrose, Lactulose, Ghatti-Gummi, Traganth; Mehlen, insbesondere Mehlen aus Weizen (Weich- oder Durum-Weizen), Roggen, Hafer, Gerste, Emmer, Dinkel, Einkorn, Buchweizen, Hirse, Mais, Reis; Stärke, insbesondere Stärke aus Weizen, Kartoffeln, Mais, Reis, Tapioka und Hafer, chemisch oder physikalisch modifizierte Stärke, abgebaute Stärke (Stärkehydrolysate) und Stärkederivaten, vorzugsweise Dextrine oder Maltodextrine, insbesondere Dextrine und Maltodextrine aus Weizen, Kartoffeln, Mais, Reis und Hafer, Cyclodextrine, Oligosaccharide, insbesondere Oligofructose; Zuckeralkoholen, insbesondere Sorbit, Mannit, Isomalt, Maltit, Maltilol-Sirup, Lactit, Xylit, Erythrit; und Mischungen aus den vorgenannten Trägerstoffen.

Ballaststoffe sind weitgehend unverdauliche Nahrungsbestandteile. Ballaststoffe in der Nahrung vergrößern allein durch ihr Vorhandensein das Nahrungsvolumen, ohne gleichzeitig auch den Energiegehalt zu steigern. Einige Ballaststoffe haben die Eigenschaft, Wasser zu binden, was dazu führt, dass sie im Magen Wasser aufnehmen. Die daraus resultierende mechanische Volumenzunahme führt zu einer stärkeren Dehnung des Magensacks, die ihrerseits zu einer Senkung des appetitanregenden Ghrelin-Spiegels und damit zu einer Zunahme des Sättigungsgefühls führt. Durch den Zusatz eines Ballaststoffes zu dem erfindungsgemäßen Kombinationsmittel lässt sich damit der Appetit zusätzlich reduzieren.

Bevorzugte Trägerstoffe sind Siliciumdioxid, Gummi arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Maisbasierende Stärken sowie Stärken aus Tapioka, Reis, Weizen oder Kartoffeln.

Die oben genannten Trägerstoffe zur Herstellung des erfindungsgemäßen Kombinationsmittels werden erfindungsgemäß entweder einzeln oder in Kombination miteinander als Mischung verwendet.

Der erfindungsgemäß in dem Kombinationsmittel einzusetzende wenigstens eine Hilfsstoff, insbesondere pharmazeutische Hilfsstoff, ist in Abhängigkeit von der Darreichungsform des Kombinationsmittels gemäß der Erfindung ausgewählt aus der Gruppe, die besteht aus: Füllstoffen, Lösungsmitteln, Befeuchtungsmitteln, Emulgatoren, Lösungsvermittler, Netzmittel, Puffer, Verdickungs- und Bindemittel, Umhüllungsmittel, Zerfallsbeschleuniger, Sprengmittel, Gleit- und Schmiermittel, Formentrennmittel, Fließregulierungsmittel, Antioxidantien, Konservierungsstoffe und Resorptionsbeschleuniger.

Derartige Hilfsstoffe sind Stoffe, die bei der Herstellung des Kombinationsmittels, insbesondere bei der Herstellung von Arzneimitteln, verwendet werden. Derartige Hilfsstoffe sind optimaler Weise pharmakologisch und toxikologisch inert und haben folgende Funktionen:
- Formgebung: Hilfsstoffe tragen den Arzneistoff und geben dem Arzneimittel seine Form;
- Herstellbarkeit: Hilfsstoffe ermöglichen oder verbessern bestimmte Fertigungsschritte in der Arzneimittelherstellung;
- Steuerung der Wirkstofffreigabe: Hilfsstoffe steuern gezielt die Freigabe von Wirkstoffen oder transportieren sie gezielt an ihren Wirkungsort; und
- Stabilitätsverbesserung: Hilfsstoffe gewährleisten die ausreichende Haltbarkeit eines Arzneimittels.

Die oben genannten Hilfsstoffe zur Herstellung des erfindungsgemäßen Kombinationsmittels werden erfindungsgemäß entweder einzeln oder in Kombination miteinander als Mischung verwendet.

**In** einer weiteren bevorzugten Ausgestaltung des ersten Erfindungsgegenstandes ist das erfindungsgemäße Kombinationsmittel dadurch gekennzeichnet, dass es umfasst oder besteht aus:
0,00001 (0,1 ppm) bis 0,001 (10 ppm) Gew.-% der einen oder mehreren erste Sättigungskomponente(n);
0,1 bis 20 Gew.-%, vorzugsweise 5 bis 20 Gew.-%, zugesetztes freies Arginin, insbesondere freies L-Arginin; und
optional
1 bis 80 Gew.-%, vorzugsweise 1 bis 95 Gew.-%, der einen oder mehreren zweite Sättigungskomponente(n); und/oder
0,00001 (0,1 ppm) bis 1 Gew.-% der einen oder mehreren sensorisch aktiven Substanz;
jeweils bezogen auf das Gesamtgewicht des Kombinationsmittels.

Besonders vorteilhaft sind Zusammensetzungen des Kombinationsmittels, in dem der Anteil an zugesetztem freien Arginin in dem Kombinationsmittel mindestens 0,5 bis 10 Gew.-% beträgt, insbesondere der Gehalt an zugesetztem freien L-Arginin mindestens 0,5 bis 10 Gew.-% beträgt, bezogen auf das Gesamtgewicht des Kombinationsmittels.

Das erfindungsgemäße Kombinationsmittel, insbesondere nach obigen Varianten, zeichnet sich dadurch aus, dass es das Hungergefühl verringert oder gegen Heißhungerattacken wirkt, ein Gefühl von Sättigung vermittelt und/oder den Stoffwechsel anregt.

Die Wirkung der erfindungsgemäßen Kombinationsmittel wurde an 32 Probanden getestet:

**Tabelle 2: Angaben zu den Testpersonen**

| Variable | Mittelwert | Standardabweichung | Bereich |
|---|---|---|---|
| Alter (Jahre) | 25,8 | 3,6 | 20 - 36 |
| Größe (m) | 1,82 | 0,1 | 1,68 - 1,93 |
| Gewicht (kg) | 89,3 | 11,2 | 69,5 - 111 |
| BMI (kg/m²) | 26,8 | 1,9 | 24,0 - 31,9 |
| Blutglucose nach dem Fasten (mg/dl) | 74,6 | 7,3 | 62 - 97 |

Zum Test wurden 32 Probanden einer medizinischen Untersuchung unterzogen und erhielten an vier unterschiedlichen Tagen Testlösungen innerhalb eines oralen Glucose-Toleranz-Tests (oGTT) als Stimulans zur Sättigung. An den Testtagen, nach einem Fasten über Nacht von 12 Stunden, bewerteten die Probanden ihr subjektives Hungergefühl an einer Visuellen Analogskala (VAS) (Skala zur Messung einer subjektiven Einstellung) und erhielten danach entweder:
▪ C (Kontrolle): 75 g Glucose plus 0,15 mg Nonivamid in 300 ml Wasser (erste Sättigungskomponente);
▪ ARG: 75 g Glucose plus 0,15 mg Nonivamid plus 1,2 g L-Arginin in in 300 ml Wasser (Kombinationsmittel gemäß der vorliegenden Erfindung);
▪ WPH: 75 g Glucose plus 0,15 mg Nonivamid plus 2 g Weizenprotein-Hydrolysat (Arginingehalt: etwa 2 bis 3 %) in 300 ml Wasser (zweite Sättigungskomponente), oder
▪ WPH + ARG: 75 g Glucose plus 0,15 mg Nonivamid plus 2 g Weizenprotein-Hydrolysat plus 1,2 g L-Arginin in 300 ml Wasser (Kombinationsmittel gemäß der vorliegenden Erfindung).

Der Nonivamid-Glucosedrink enthielt außerdem 150 mg ¹³C-Na-Acetat in 300 ml Wasser und 15 µl Ethanol.

Parallel dazu wurden von den Probanden Atemproben entnommen, da die Testlösungen mit ¹³C-Na-acetat markiert war, einem Marker, um das Entleeren des Magens nachzuweisen. Innerhalb der nächsten 140 Minuten wurden jeweils 6 Blutproben und 11 Atemproben entnommen und analysiert. Bevor die Probanden ein standardisiertes ad libitum Frühstück einnahmen, um die Nahrungsaufnahme zu analysieren, bewerteten die Probanden erneut ihr subjektives Hungergefühl an einer Visuellen Analog-Skala (VAS).

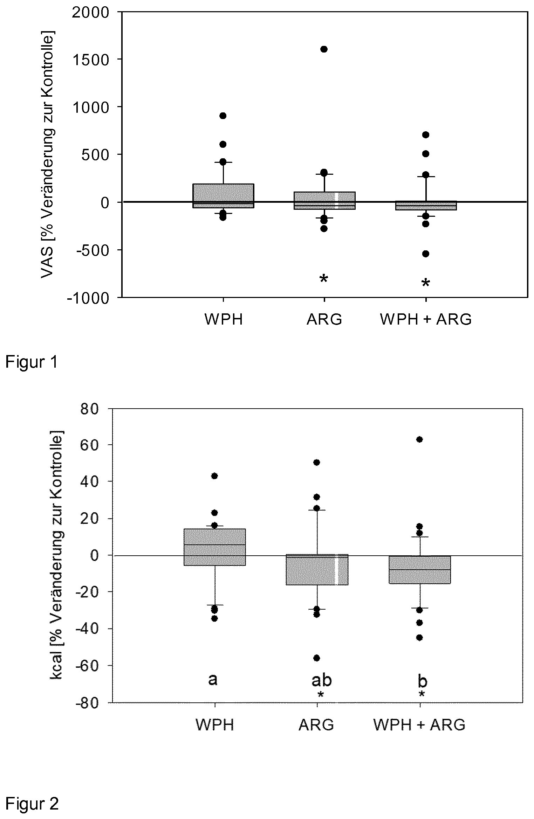

Die Testergebnisse sind in den Figuren 1 bis 4 wiedergegeben.

Wie aus Figur 1 ersichtlich ist, war das Hungergefühl nach Verabreichung der erfindungsgemäßen Kombinationsmittel (ARG und WPH + ARG) signifikant geringer als bei der Kontrolle (als Linie dargestellt) und der nicht erfindungsgemäßen Zusammensetzung WPH. Am wenigstens war das Hungergefühl ausgeprägt nach Verabreichung des erfindungsgemäßen Kombinationsmittels (WPH + ARG).

Wie aus Figur 2 ersichtlich ist, war die *Ad libitum* Energieaufnahme 140 min nach Verabreichung der erfindungsgemäßen Kombinationsmittel (ARG und WPH + ARG) signifikant geringer als bei der Kontrolle (als Linie dargestellt) und der nicht erfindungsgemäßen Zusammensetzung WPH. Die Verabreichung des erfindungsgemäßen Kombinationsmittels (WPH + ARG) führte dazu, dass die ad *libitum* Energieaufnahme am geringsten war.

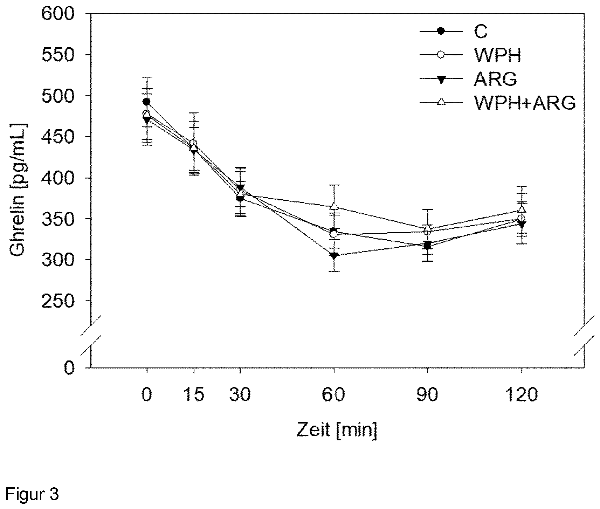

Wie aus Figur 3 ersichtlich ist, hat das erfindungsgemäßen Kombinationsmittel (WPH + ARG) keinen Einfluss auf das Hungerhormon Ghrelin.

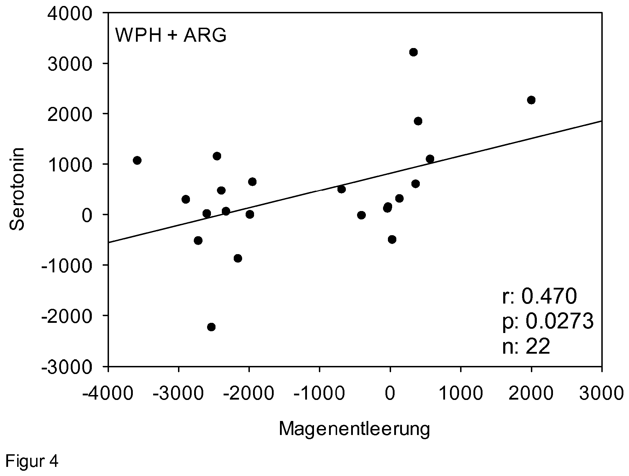

In Figur 4 zeigt eine Korrelationsanalyse, dass Serotonin mit der Magenentleerung nach Verabreichung des erfindungsgemäßen Kombinationsmittels (WPH + ARG) korreliert, sodass ein Anstieg der Sättigung angenommen werden kann.

Wie eine Untersuchung des Plasmaglucoselevels ergab, ist der Glucosespiegel im Plasma nach Verabreichung der erfindungsgemäßen Kombinationsmittel (ARG und WPH + ARG) deutlich niedriger als bei der Kontrolle und der nicht erfindungsgemäßen Zusammensetzung WPH. Die besten Ergebnisse wurden erzielt für das Kombinationsmittel, das eine zweite Sättigungskomponente enthielt (WPH + ARG).

**Tabelle 3:**

| Zeit [min] | Kontrolle Δ [mg/dL] | WPH Δ [mg/dL] | ARG Δ [mg/dL] | WPH + ARG Δ [mg/dL] |
|---|---|---|---|---|
| 0 | 0 | 0 | 0 | 0 |
| 15 | 28,42 ± 3,62 | 43,65 ± 4,20 | 34,14 ± 6,51 | 25,78 ± 3,90 |
| 30 | 44,62 ± 4,42 | 55,08 ± 5,01 | 42,21 ± 5,11 | 53,80 ± 5,57 |
| 60 | 20,15 ± 7,47 | 24,48 ± 6,66 | 17,56 ± 9,40 | 25,34 ± 6,59 |
| 90 | 5,37 ± 6,58 | 6,50 ± 6,03 | -2,80 ± 6,28 | 0,98 ± 4,31 |
| 120 | -15,40 ± 5,11 | -6,46 ± 4,40 | -18,66 ± 4,46 | -10,81 ± 4,77 |

Aus den o.g. Werten lässt sich rückschließen, dass eine regelmäßige Anwendung des erfindungsgemäßen Kombinationsmittels den Anstieg des glykämischen Index und seine negativen metabolischen Konsequenzen vermindern kann.

**Tabelle 4: Nachweis von ¹³CO₂ im Atem als Marker für die Magenentleerung**

| **Behandlung** | **Δ Änderung der Magenentleerung gegenüber der Kontrolle [Behandlung - Kontrolle]** | **t-Test vs. Kontrolle p-Wert** | **Mechanisches Sättigungssignal** |
|---|---|---|---|
| WPH* | -791,7 ± 367,6 | 0,04 | ↑ steigt signifikant = keine |
| ARG | -634,4 ± 398,2 | 0,12 | Veränderung |
| WPH+ARG* | -1129,0 ± 323,9 | 0,001 | ↑ steigt signifikant |

Das Prinzip dieser Untersuchung beruht darauf, dass die Testlösungen mit ¹³C-Na-Acetat markiert wurden. ¹³C-Na-Acetat wird im Magen nicht absorbiert, sondern erst im Dünndarm. In der Leber erfolgt eine Metabolisierung, so dass anschließend ¹³CO₂ über die Lunge abgeatmet wird. Anhand der ¹³CO₂-Konzentration lässt sich rückschließen, ob die Testlösungen bereits den Magen verlassen haben. Je höher die Konzentration des ¹³CO₂ in der Atemluft, umso schneller ist die Magenentleerung. Zur Berechnung wurde der basale ¹²CO₂ Wert sowie die Körperoberfläche mittels DuBois-Formel eines jeden Probanden berücksichtigt.

**Tabelle 5: Nachweis des Sättigungshormons Serotonin nach Verabreichung des Weizenproteins allein und in Kombination mit Arginin.**

| **Behandlung** | **Δ Änderung von Serotonin gegenüber der Kontrolle [Behandlung - Kontrolle]** | **t-Test vs. Kontrolle p-Wert** | **Hormonelles Sättigungssignal** |
|---|---|---|---|
| WPH | -28,1 ± 154,9 | 0,813 | - |
| ARG | 283,9 ± 189,8 | 0,222 | - |
| WPH+ARG* | 350,4 ± 218,2 | 0,007 | ↑ steigt signifikant |

Wie die obigen Untersuchungen eindeutig belegen, führt die Verabreichung eines erfindungsgemäßen Kombinationsmittels zu einer Verringerung des Appetits und zur Vermittlung eines rascheren Sättigungsgefühls und folglich zu einer Reduzierung der Energieaufnahme

Das erfindungsgemäße Kombinationsmittel, das neben den erfindungsgemäß zu verwendenden Bestandteilen (a) bis (d) noch einen oder mehrere Trägerstoffe oder Hilfsmittel umfassen kann, kann beispielsweise durch mechanische Mischvorgänge, wobei gleichzeitig auch eine Zerkleinerung der Partikel erfolgen kann, oder mittels Sprühtrocknung hergestellt werden. Bevorzugt ist ein erfindungsgemäßes Kombinationsmittel, das feste Trägerstoffe oder Hilfsstoffe umfasst und mittels Sprühtrocknung hergestellt wird. Alternativ können sowohl die eine oder die mehreren erste Sättigungskomponente(n) (Bestandteil (a)) als auch die eine oder die mehreren zugesetzte(n) freie(n) Aminosäure(n) (Bestandteil (b)) als Lösungen zubereitet und der einen oder den mehreren zweite Sättigungskomponente(n) (Bestandteil (c)) zugesetzt werden. Die Aromastoffe (Bestandteil (d)) können als solche oder in einer eigenständigen, dem Fachmann wohlbekannten Zubereitung als Lösung oder auf Träger aufgezogen eingesetzt werden.

Bevorzugte erfindungsgemäße, Trägerstoffe umfassende Kombinationsmittel, die mittels Sprühtrocknung hergestellt wurden, besitzen eine mittlere Partikelgröße im Bereich von 3 bis 1000 µm und eine Restfeuchte von kleiner oder gleich 5 Gew.-%.

Ein zweiter Erfindungsgegenstand und Aspekt betrifft das erfindungsgemäße Kombinationsmittel zur medizinischen Anwendung, d.h. zur Anwendung in einem therapeutischen Verfahren, insbesondere als Mittel zur Verringerung des Appetits und/oder als Mittel zur Vermittlung eines Gefühls von Sättigung, und damit einhergehend zur Reduzierung der kalorischen Aufnahme. Das erfindungsgemäße Kombinationsmittel ist daher bevorzugt ein Medikament bzw. wird zur Verwendung als Medikament eingesetzt.

In dem therapeutischen Verfahren wird das erfindungsgemäße Kombinationsmittel derart eingesetzt, dass es den Appetit reduziert und/oder ein Gefühl von Sättigung hervorruft und somit zu einer Reduzierung der kalorischen Aufnahme führt. Bevorzugt wird nach einer Variante des zweiten Aspekts das Kombinationsmittel daher verwendet zur Behandlung von Übergewicht oder Adipositas

Durch die Reduzierung der kalorischen Aufnahme, die aus einem verringerten Appetit und einem schnelleren Sättigungsgefühl resultiert, eignet sich das erfindungsgemäße Kombinationsmittel insbesondere zur therapeutischen Reduktion des Gewichtes und somit zur therapeutischen Behandlung von Übergewicht und Adipositas. Bei Adipositas handelt es sich um eine Ernährungs- und Stoffwechselkrankheit mit starkem Übergewicht, die durch eine über das normale Maß hinausgehende Vermehrung des Körperfettes gekennzeichnet ist.

In wissenschaftlichen Studien konnte gezeigt werden, dass Übergewicht im direkten Zusammenhang stehen mit kardiovaskulären Erkrankungen, Schlaganfall, Diabetes sowie Gelenkschäden und Verschleiß der Wirbelsäule.

Durch die Reduzierung der kalorischen Aufnahme und damit einhergehend Verringerung des Körpergewichts eignet sich das erfindungsgemäße Kombinationsmittel vorteilhafter Weise auch zur Prävention von kardiovaskulären Erkrankungen, insbesondere Bluthochdruck, Diabetes, insbesondere Typ 2 Diabetes Mellitus, Schlaganfall sowie Gelenkschäden.

In einer weiteren bevorzugten Variante ist das Kombinationsmittel verwendet zur Prävention von kardiovaskulären Erkrankungen, Schlaganfall, Diabetes sowie Gelenkschäden und Verschleiß der Wirbelsäule.

Der zweite Erfindungsgegenstand und Aspekt betrifft weiter das erfindungsgemäße Kombinationsmittel zur nicht-therapeutischen Anwendung, insbesondere als Mittel zur Reduzierung des Appetits und/oder als Mittel zur Vermittlung eines Gefühls von Sättigung, und damit einhergehend zur Reduzierung der kalorischen Aufnahme.

In dem nicht-therapeutischen Verfahren wird das erfindungsgemäße Kombinationsmittel derart eingesetzt, dass es den Appetit reduziert und/oder ein Gefühl von Sättigung hervorruft und somit zu einer reduzierten kalorischen Aufnahme führt.

Durch die Reduzierung der kalorischen Aufnahme eignet sich das erfindungsgemäße Kombinationsmittel vorteilhafter Weise zur kosmetischen Reduktion des Gewichtes oder bei der Prophylaxe vor Übergewicht.

In einer am meisten bevorzugten Ausführungsform gemäß einem weiteren Aspekt der vorliegenden Erfindung ist das Kombinationsmittel ein Nahrungsergänzungsmittel oder ein Arzneimittel. Das Nahrungsergänzungsmittel oder Arzneimittel kann in Form von Pulvern, Feststoffmischungen, Granulaten, Pellets, Kapseln, Tabletten (nicht überzogene sowie überzogene Tabletten), Dragees, Lösungen, Dispersionen in flüssigen Phasen, Emulsionen, Pasten oder als andere schluck- oder kaubare Zubereitungen vorliegen.

Der dritte Erfindungsgegenstand und Aspekt der vorliegenden Erfindung betrifft die Verwendung des erfindungsgemäßen Kombinationsmittels als Mittel zur nicht-therapeutischen
(i) Reduzierung des Appetits; und/oder
(ii) Vermittlung eines Sättigungsgefühls; und/oder
(iii) Reduzierung der Energieaufnahme; und/oder
(iv) Reduzierung des Körpergewichts.

Um eine Reduktion des Körpergewichts bei einem Patienten oder einem Konsumenten zur unterstützen, hat es sich als sinnvoll erwiesen, die kalorische Aufnahme zu begrenzen. Dies kann erfolgt dadurch, dass mit der oralen Aufnahme des erfindungsgemäßen Kombinationsmittels der Appetit verringert und ein schnelleres Gefühl von Sättigung vermittelt wird. Dadurch verzehrt ein Patient oder Konsument weniger, wodurch die Energieaufnahme verringert wird.

Wie durch die oben beschriebenen Untersuchungen nachgewiesen, lässt sich mit dem erfindungsgemäßen Kombinationsmittel der Appetit reduzieren und/oder ein schnelleres Sättigungsgefühl vermitteln. Aufgrund dieser Eigenschaften eignet sich das Kombinationsmittel hervorragend als Mittel zur Reduzierung der Energieaufnahme und/oder somit zur Reduzierung des Körpergewichts.

Gemäß einem weiteren Aspekt betrifft die vorliegende Erfindung die Verwendung des erfindungsgemäßen Kombinationsmittels zur Herstellung einer oral konsumierbaren Zubereitung oder einer Lebensmittelzutat.

Oral konsumierbare Zubereitungen, insbesondere Lebensmittel, Nahrungsergänzungsmittel, Futtermittel oder Arzneimittel, sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel) oder wieder aus der Mundhöhle entfernt zu werden (z.B. Kaugummis) Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmitteln, Futtermitteln und Arzneimitteln) bei ihrer Herstellung, Verarbeitung oder Bearbeitung zugesetzt werden und dazu vorgesehen sind, in die menschliche oder tierische Mundhöhle eingebracht zu werden.

Hierunter fallen insbesondere (kalorienreduzierte) Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Insekten oder Insektenprodukten, Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie, bevorzugt aber proteinreiche Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte mit hohem Anteil veganer, bevorzugt pflanzlicher Proteine, z.B. Algen-, Weizen-, Raps-, Sonnenblumen-, Reis-, Kartoffel-, Mais-, Soja- , Bohnen-, Erbsen-, Linsen-, Lupinen-, Erdnuss-, Alfalfa-Proteinisolaten, Proteinfraktionen, partiellen oder vollständigen Proteinhydrolysaten, oder physikalisch-chemisch, enzymatisch oder fermentativ veränderten Proteinen der vorgenannten Quellen, so z.B. aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fleischersatzprodukte aus vorgenannten vegetarischen oder veganen Proteinquellen, Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffel-chips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen.

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte mit hohem Anteil veganer, bevorzugt pflanzlicher Proteine, z.B. Algen-, Weizen-, Raps-, Sonnenblumen-, Reis-, Kartoffel-, Mais-, Soja- , Bohnen-, Erbsen-, Linsen-, Lupinen-, Erdnuss-, Alfalfa-Proteinisolaten, Proteinfraktionen, partiellen oder vollständigen Proteinhydrolysaten, oder physikalisch-chemisch, enzymatisch oder fermentativ veränderten Proteinen der vorgenannten Quellen, Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, -tabletten oder -sachets, sonstige Zubereitungen zum Süßen oder Weißen von Getränken oder anderen Nahrungsmitteln, Gemüsezubereitungen (z.B. Ketchup, Soßen, Trockengemüse, Tiefkühlgemüse, vorgegartes Gemüse, eingekochtes Gemüse), Fertiggerichte und Suppen (z.B. Trockensuppen, Instant-Suppen, vorgegarte Suppen) Fleischersatzprodukte aus vorgenannten vegetarischen oder veganen Proteinquellen.

Unter einer Lebensmittelzutat ist im Kontext der vorliegenden Erfindung ein oral konsumierbarer Stoff oder ein oral konsumierbares Produkt zu verstehen, welches als Bestandteil und gegebenenfalls unter Anwendung weiterer Prozessschritte zur Herstellung einer der Ernährung oder dem Genuss dienenden gebrauchs- oder verzehrfertigen Zubereitung (insbesondere eines Lebensmittels, eines Nahrungsergänzungsmittels oder eines Futtermittels) verwendet wird.

Weitere Bestandteile einer der Ernährung oder dem Genuss dienenden oral konsumierbaren Zubereitung können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbin-säure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Die Energiedichte eines oral konsumierbaren Produktes (insbesondere eines Lebensmittels, eines Nahrungsergänzungsmittels, eines Futtermittels oder eines Arzneimittels) lässt sich senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden.

Darüber hinaus kann die Energiedichte eines oral konsumierbaren Produktes durch Ballaststoffe weiter gesenkt werden. Ballaststoffe sind weitgehend unverdauliche Nahrungsbestandteile. Ballaststoffe in der Nahrung vergrößern allein durch ihr Vorhandensein das Nahrungsvolumen, ohne gleichzeitig auch den Energiegehalt zu steigern. Einige Ballaststoffe haben die Eigenschaft, Wasser zu binden, was dazu führt, dass sie im Magen Wasser aufnehmen. Die daraus resultierende mechanische Volumenzunahme führt zu einer stärkeren Dehnung des Magensacks, die ihrerseits zu einer Senkung des appetitanregenden Ghrelin-Spiegels und damit zu einer Zunahme des Sättigungsgefühls führt. Damit lässt sich gleichzeitig der Appetit reduzieren. Vorzugsweise ist ein erfindungsgemäß anzuwendendes oral konsumierbares Produkt ausgewählt aus der Gruppe bestehend aus Süßwaren, vorzugsweise kalorienreduzierte bzw. kalorienfreie Süßwaren, vorzugsweise ausgewählt aus der Gruppe bestehend aus Müsliriegelprodukte, Fruchtgummi, Dragees, Hartkaramellen und Kaugummi,
- nicht-alkoholische Getränke, vorzugsweise ausgewählt aus der Gruppe bestehend aus Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- und Gemüsesaftzubereitungen,
- Instantgetränke, vorzugsweise ausgewählt aus der Gruppe bestehend aus Instant-(Grün-, Schwarz-, Rooibos-, Kräuter-)Tee-Getränke,
- Getreideprodukte, vorzugsweise ausgewählt aus der Gruppe bestehend aus zuckerarmen und -freien Frühstückscerealien und Müsliriegeln,
- Milchprodukte, vorzugsweise ausgewählt aus der Gruppe bestehend aus fettreduzierten und fettfreien Milchgetränken, Joghurt, Kefir, Molke, Buttermilch und Eiscreme,
- Produkte aus Sojaprotein oder anderen Sojabohnen-Fraktionen, vorzugsweise ausgewählt aus der Gruppe bestehend aus Sojamilch, aus Sojamilch gefertigten Produkten, Getränken enthaltend isoliertes oder enzymatisch behandeltes Sojaprotein, Sojamehl enthaltenden Getränken, Sojalecithin-haltigen Zubereitungen, aus Sojalecithin-haltigen Zubereitungen gefertigten Produkten und Mischungen mit Fruchtzubereitungen und ggf. Aromen,
- Produkte aus anderen pflanzlichen Proteinen bzw. Proteinfraktionen, wobei die Quelle für die Proteine bevorzugt ausgewählt aus der Gruppe bestehend aus Süßlupine, Erbse, Bohne, Linse, Weizen, Raps, Algen, Kartoffeln und Reis,
- Süßstoffzubereitungen, -tabletten und -sachets
- zuckerfreie Dragees,
- Eiscreme, mit oder ohne Bestandteile auf Basis von Milch, vorzugsweise zuckerfrei.
- herzhafte ("savory") Lebensmittel, vorzugsweise ausgewählt aus der Gruppe bestehend aus Fleischersatzprodukten, Nudelgerichten, Suppen, proteinreichen Suppen, etc.

Gemäß einem weiteren Aspekt betrifft die vorliegende Erfindung eine oral konsumierbare Zubereitung oder eine Lebensmittelzutat, welche das erfindungsgemäße Kombinationsmittel umfasst.

In einer bevorzugten Variante umfasst die erfindungsgemäß oral konsumierbare Zubereitung oder eine Lebensmittelzutat das erfindungsgemäße Kombinationsmittel in Kombination mit einem oder mehreren Ballaststoff(en).

In einer derartigen oral konsumierbaren Zubereitung oder Lebensmittel ist das Kombinationsmittel in einer Menge von 1 bis 80 Gew.-% enthalten, bevorzugt 3 bis 50 %, besonders bevorzugt 5 bis 30 % jeweils bezogen auf das Gesamtgewicht der oral konsumierbaren Zubereitung.

### Ausführungsbeispiele

### Anwendungsbeispiel 1: Fettarme Joghurts

Zur Herstellung der Joghurts werden die Inhaltsstoffe nach u.g. Rezepturen gemischt und auf 5 °C gekühlt.

| | Zubereitung (Angaben als Gew.-%) | | | |
|---|---|---|---|---|
| Inhaltsstoff | A | B | C | D |
| Sucrose | 10 | 8 | 6 | - |
| Rebaudiosid A 98 % | - | - | - | 0,050 |
| Extrakt aus Rubus suavissimus, enthaltend 20 Gew.-% Rubusosid, bezogen auf das Gesamtgewicht des Extraktes, z.B. von PlantExtrakt | - | 0,010 | 0,010 | - |
| Erdbeeraroma | 0,1 | 0,1 | 0,1 | 0,1 |
| Hesperetin | - | 0,001 | 0,001 | 0,002 |
| Phloretin | - | - | 0,002 | 0,002 |
| L-Arginin | 1,5 | 1,5 | 2 | 2,5 |
| Capsaicin | 0,00001* | - | - | 0,00001 |
| trans-Pellitorin | - | 0,0001* | - | - |
| Nonivamid | - | - | 0,00001 | 0,00001 |
| Joghurt (0,1 % Fett, 3 % Protein) | ad 100 | | | |

| | | | | |
|---|---|---|---|---|
| * außerhalb des Schutzbereichs der vorliegenden Erfindung | | | | |

### Anwendungsbeispiel 2: Proteinmixgetränke auf Trockenbasis

Die Zutaten werden nach u.g. Rezepturen gemischt und unter Schutzgas oder im Vakuumbeutel abgepackt. Zur Verwendung wird das Pulver in 100 ml Wasser (Zimmertemperatur) aufgerührt und dann direkt verzehrt.

| Inhaltsstoff | Einsatz in Gramm | | | | | | |
|---|---|---|---|---|---|---|---|
| Zubereitung | A | B | C | D | E | F | G |
| Zucker, fein | 5 | 5 | 5 | 5 | 5 | 5 | 5 |
| Nonivamid, 0,1 %ig in Alkohol | 0,03 | 0,015 | 0,03 | 0,015 | 0,03 | 0,03 | 0,015 |
| trans-Pellitorin, 1 %ig in 1,2-Propylenglycol/Diethylmalonat | - | 0,015 | - | 0,015 | - | - | 0,01 |
| L-Arginin | 5 | 10 | 5 | 2 | 10 | 15 | 5 |
| Aroma Typ Cappuccino, sprühgetrocknet | 0,2 | 0,2 | 0,2 | 0,2 | - | - | - |
| Aroma Typ Erdbeer, sprühgetrocknet | - | - | - | - | 0,1 | 0,1 | 0,1 |
| Rote Bete Farbpulver, sprühgetrocknet | - | - | - | - | 0,1 | 0,08 | 0,1 |
| Zuckercouleur | 0,15 | 0,15 | 0,15 | 0,15 | - | - | - |
| Xanthan | 0,1 | 0,1 | 0,1 | 0,1 | 0,1 | 0,1 | 0,1 |
| Magermilchpulver | 10 | - | - | - | 10 | - | - |
| Milchpulver lactosereduziert (Fa. Omira, Ravensburg) | - | - | - | - | - | 10 | - |
| Lupinenproteinpulver | - | - | - | 5 | - | - | - |
| Sojaproteinhydrolysat (60 % Hydrolysegrad) | - | 10 | - | - | - | - | - |
| Weizenprotein-hydrolysat (80 % Hydrolysegrad) | - | - | - | - | - | - | 10 |
| Rapsproteinn (partiell hydrolysiert, Napinreich) | - | - | 10 | - | - | - | - |

### Anwendungsbeispiel 3: Molkenproteingetränk

Herstellung eines Frucht-Molkenproteingetränkes nach u.g. Rezepturen mit anschließender Homogenisierung und Pasteurisierung.

| Inhaltsstoff | Einsatz in Gew.-% | |
|---|---|---|
| | A | B |
| Molkenproteinisolat (min. 88 % Protein, bezogen auf die Trockensubstanz) | 8 | 8 |
| Fruchtsaftkonzentrat-Mix (Mango, Banane, Karotte, Orange) | 10 | 10 |
| Zitronensäure 50%ig | 0,2 | 0,2 |
| Pektin | 0,4 | 0,4 |
| Vitamin-Mischung | 0,01 | 0,01 |
| Mineralsalz-Mischung | 1 | 1 |
| Nonivamid | 0,0003 | 0,00015 |
| L-Arginin | 2 | 3 |
| Mango Aroma (Symrise) | 0,05 | 0,05 |
| Trinkwasser | ad 100 | ad 100 |

### Anwendungsbeispiel 4: Proteinriegel

Die Cerealien und das Proteinisolat werden gemischt. Aus den restlichen Zutaten (ohne Aroma) wird ein Sirup gekocht. Am Ende der Kochzeit wird das Aroma zugegeben. Der Sirup wird mit den Cerealien gemischt und zu Riegeln geformt.

| Inhaltsstoff | Einsatz in Gew.-% | | |
|---|---|---|---|
| | A | B | C |
| Haferflocken | 13 | 13 | 13 |
| Reis-Crispies | 14 | 14 | 14 |
| Corn Flakes | 10 | 10 | 10 |
| Molkenproteinisolat (min. 88 % Protein, bezogen auf die Trockensubstanz) | 10 | - | 10 |
| Erbsenproteinisolat (min. 80 % Protein, bezogen auf die Trockensubstanz, 10 % Hydrolysegrad) | - | 10 | - |
| Glucose Sirup | 0,1 | 0,1 | 0,1 |
| Saccharose | 12 | 12 | 12 |
| Glycerin | 1 | 1 | 1 |
| Salz | 0,2 | 0,2 | 0,2 |
| Trinkwasser | 13,5 | 13,5 | 13,5 |
| Pflanzenfett | 10 | 10 | 10 |
| Lecithin | 0,2 | 0,2 | 0,2 |
| Bittermasking Aroma (Symrise) | 0,3 | 0,3 | 0,3 |
| Karamel Aroma (Symrise) | 0,1 | 0,1 | 0,1 |
| Nonivamid | 0,00025 | 0,0005 | 0,0004 |
| L-Arginin | 5 | 4 | 3 |

## Patentansprüche

1. Kombinationsmittel umfassend oder bestehend aus:
(a) eine oder mehrere erste Sättigungskomponente(n), wobei die eine oder die mehreren erste(n) Sättigungskomponente(n) ausgewählt ist/sind aus der Gruppe, bestehend aus Nonivamid, Pellitorin, Cinnamylalkohol-Derivaten, ausgewählt aus der Gruppe, bestehend aus Cinnamylformiat, Cinnamylacetat, Cinnamylpropionat, Cinnamylbutyrat, Cinnamylisobutyrat, Cinnamylisovalerianat, Cinnamyltiglinat, Cinnamylbenzoat, Cinnamylphenylacetat, und Cinnamylcinnamat, Zimtsäureamid-Derivaten der allgemeinen Formel 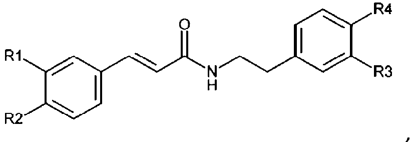 wobei R1, R2, R3 und R4 unabhängig voneinander für Wasserstoff oder eine Methoxygruppe stehen, aromatischen Alkensäure-Derivaten, ausgewählt aus der Gruppe, bestehend aus Dihydropiperlinguminin, Chingchengenamid A, Piperdardin und Methyl-7-(1,3-benzodioxol-5-yl)hepta-2,4-dienoat, und Mischungen daraus;
(b) zugesetztes freies Arginin oder dessen möglichen Stereoisomeren und Mischungen der Stereoisomeren oder dessen Natrium-, Calcium-, Magnesium-, Kalium- oder Ammoniumsalzen; und
optional
(c) eine oder mehrere zweite Sättigungskomponente(n), wobei die eine oder die mehreren zweite(n) Sättigungskomponente(n) ausgewählt ist aus der Gruppe, bestehend aus pflanzlichen oder tierischen Proteinen und Proteinhydrolysaten; und/oder
(d) eine oder mehrere sensorisch aktive Substanz(en).

2. Kombinationsmittel nach Anspruch 1, wobei die eine oder die mehreren erste(n) Sättigungskomponente(n) ausgewählt ist/sind aus der Gruppe, die besteht aus Nonivamid, Pellitorin, und Mischungen daraus.

3. Kombinationsmittel nach einem der vorangehenden Ansprüche, wobei die eine oder die mehreren zweite(n) Sättigungskomponente(n) Protein(e) ausgewählt ist/sind aus der Gruppe, die besteht aus Proteinen aus Fleisch (Säugetiere, Vögel, Reptilien, Amphibien, Fisch), Krebsen, Schalentieren, Muscheln, Weichtieren, Insekten, Eiern, Milch, insbesondere Casein und Molke, Algen, Getreide, insbesondere Weizen, Gerste, Raps, Sonnenblumen, Reis, Kartoffeln, Mais, Soja, Bohnen, Erbsen, Linsen, Lupinen, Erdnuss, Alfalfa, Hanf und weiteren Proteinen aus essbaren Pflanzen, insbesondere Proteinen mit intrinsisch hohem L-Arginingehalt.

4. Kombinationsmittel nach einem der vorangehenden Ansprüche, worin die eine oder die mehreren zweite Sättigungskomponente(n) Proteinhydrolysat(e) ausgewählt ist/sind aus der Gruppe, die besteht aus Teilhydrolysat oder vollständigem Hydrolysat von Proteinen aus Fleisch (Säugetiere, Vögel, Reptilien, Amphibien, Fisch), Krebsen, Schalentieren, Muscheln, Weichtieren, Insekten, Eiern, Milch, insbesondere Casein und Molke, Algen, Getreide, insbesondere Weizen, Gerste, Raps, Sonnenblumen, Reis, Kartoffeln, Mais, Soja, Bohnen, Erbsen, Linsen, Lupinen, Erdnuss, Alfalfa, Hanf und weiteren Proteinen aus essbaren Pflanzen, insbesondere Proteinen mit intrinsisch hohem L-Arginingehalt.

5. Kombinationsmittel nach einem der vorangehenden Ansprüche, worin der Gesamtgehalt an L-Arginin in dem erfindungsgemäßen Kombinationsmittel, das die Bestandteile (a) und (b) oder die Bestandteile (a), (b) und (c) umfasst, in einer regulär konsumierbaren Menge (Portionsgröße) mindestens 0,1 g L-Arginin, bevorzugt mindestens 0,5 g L-Arginin und am meisten bevorzugt mindestens 1 g L-Arginin beträgt, bezogen auf das Gesamtgewicht der regulär konsumierbaren Menge (Portionsgröße) des Kombinationsmittels.

6. Kombinationsmittel nach einem der vorangehenden Ansprüche, welches darüber hinaus weiter umfasst:
(e) einen Trägerstoff und/oder ein Hilfsmittel bzw. Adjuvans.

7. Kombinationsmittel nach einem der vorangehenden Ansprüche, umfassend oder bestehend aus:
0,00001 Gew.-% (0,1 ppm) bis 0,001 Gew.-% (10 ppm) der einen oder mehreren erste(n) Sättigungskomponente(n);
5 bis 20 Gew.-% an zugesetztem freien Arginin; und
optional
1 bis 95 Gew.-% der einen oder mehreren zweite(n) Sättigungskomponente(n); und/oder
0,00001 Gew.-% (0,1 ppm) bis 1 Gew.-% der einen oder mehreren sensorisch aktiven Substanz(en);
jeweils bezogen auf das Gesamtgewicht des Kombinationsmittels.

8. Kombinationsmittel nach einem der Ansprüche 1 bis 7 zur medizinischen Anwendung.

9. Kombinationsmittel nach einem der Ansprüche 1 bis 7 zur Verwendung zur Behandlung von Übergewicht und Adipositas.

10. Kombinationsmittel nach einem der Ansprüche 1 bis 7 zur Verwendung zur Prävention von kardiovaskulären Erkrankungen, Schlaganfall, Diabetes sowie Gelenkschäden und Verschleiß der Wirbelsäule.

11. Kombinationsmittel nach einem der Ansprüche 1 bis 7 zur nicht-therapeutischen Anwendung.

12. Verwendung des Kombinationsmittels nach einem der Ansprüche 1 bis 7 als Mittel zur nicht-therapeutischen
(i) Reduzierung des Appetits; und/oder
(ii) Vermittlung eines Sättigungsgefühls; und/oder
(iii) Reduzierung der Energieaufnahme; und/oder
(iv) Reduzierung des Körpergewichts.

13. Verwendung des Kombinationsmittels nach einem der Ansprüche 1 bis 7 zur Herstellung einer oral konsumierbaren Zubereitung oder einer Lebensmittelzutat.

14. Oral konsumierbare Zubereitung oder Lebensmittelzutat, umfassend das Kombinationsmittel nach einem der Ansprüche 1 bis 7.

15. Oral konsumierbare Zubereitung oder Lebensmittelzutat nach Anspruch 14, welche das Kombinationsmittel nach einem der Ansprüche 1 bis 7 in einer Menge von 1 bis 80 Gew.-% umfasst, bezogen auf das Gesamtgewicht der oral konsumierbaren Zubereitung oder der Lebensmittelzutat.

## Claims

1. Combination remedy, comprising or consisting of:
(a) one or more first satiating component(s), the one or more first satiating component(s) being selected from the group consisting of nonivamide, pellitorine, cinnamyl alcohol derivatives selected from the group consisting of cinnamyl formate, cinnamyl acetate, cinnamyl propionate, cinnamyl butyrate, cinnamyl isobutyrate, cinnamyl isovalerianate, cinnamyl tiglinate, cinnamyl benzoate, cinnamyl phenyl acetate, and cinnamyl cinnamate, cinnamamide derivatives of general formula 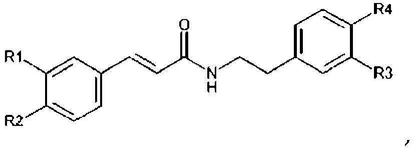 in which R1, R2, R3 and R4, independently of each other, represent hydrogen or a methoxy group, aromatic alkenoic acid derivatives selected from the group consisting of dihydropiperlonguminine, chingchengenamide A, piperdardine, and methyl-7-(1,3-benzodioxol-5-yl)hepta-2,4-dienoate, and mixtures thereof;
(b) added free arginine or possible stereoisomers thereof, and mixtures of the stereoisomers or the sodium, calcium, magnesium, potassium or ammonium salts thereof; and
optionally
(c) one or more second satiating component(s), the one or more second satiating component(s) being selected from the group consisting of plant or animal proteins and protein hydrolysates; and/or
(d) one or more sensory active substance(s).

2. Combination remedy according to claim 1, wherein the one or more first satiating component(s) is/are selected from the group consisting of nonivamide, pellitorine, and mixtures thereof.

3. Combination remedy according to any one of the preceding claims, wherein the one or more second satiating component(s) is/are protein(s) selected from the group consisting of proteins from meat (mammals, birds, reptiles, amphibians, fish), crustaceans, shellfish, mussels, mollusks, insects, eggs, milk, in particular casein and whey, algae, cereals, in particular wheat, barley, rapeseed, sunflower, rice, potatoes, corn, soybean, beans, peas, lentils, lupins, peanut, alfalfa, hemp, and other proteins from edible plants, in particular proteins with an intrinsically high L-arginine content.

4. Combination remedy according to any one of the preceding claims, wherein the one or more second satiating component(s) is/are protein hydrolysate(s) selected from the group consisting of partial hydrolysate or complete hydrolysate of proteins from meat (mammals, birds, reptiles, amphibians, fish), crustaceans, shellfish, mussels, mollusks, insects, eggs, milk, in particular casein and whey, algae, cereals, in particular wheat, barley, rapeseed, sunflower, rice, potatoes, corn, soybean, beans, peas, lentils, lupins, peanut, alfalfa, hemp, and other proteins from edible plants, in particular proteins with an intrinsically high L-arginine content.

5. Combination remedy according to any one of the preceding claims, wherein the total L-arginine content in a regularly consumable amount (portion size) of the combination remedy according to the invention comprising components (a) and (b) or components (a), (b) and (c) is at least 0.1 g of L-arginine, preferably at least 0.5 g of L-arginine, and most preferably at least 1 g of L-arginine, based on the total weight of the regularly consumable amount (portion size) of the combination remedy.

6. Combination remedy according to any one of the preceding claims, which additionally further comprises:
(e) a carrier and/or an excipient or adjuvant.

7. Combination remedy according to any one of the preceding claims, comprising or consisting of:
0.00001% by weight (0.1 ppm) to 0.001% by weight (10 ppm) of the one or more first satiating component(s);
5 to 20% by weight of added free arginine; and
optionally
1 to 95% by weight of the one or more second satiating component(s); and/or
0.00001% by weight (0.1 ppm) to 1% by weight of the one or more sensory active substance(s);
in each case based on the total weight of the combination remedy.

8. Combination remedy according to any one of claims 1 to 7 for medical use.

9. Combination remedy according to any one of claims 1 to 7 for use in the treatment of excess weight and obesity.

10. Combination remedy according to any one of claims 1 to 7 for use in the prevention of cardiovascular diseases, stroke, diabetes, joint damage, and degeneration of the spine.

11. Combination remedy according to any one of claims 1 to 7 for non-therapeutic use.

12. Use of the combination remedy according to any one of claims 1 to 7 as an agent for non-therapeutically
(i) reducing appetite; and/or
(ii) imparting a feeling of satiety; and/or
(iii) reducing energy intake; and/or
(iv) reducing body weight.

13. Use of the combination remedy according to any one of claims 1 to 7 for producing an orally consumable preparation or a food ingredient.

14. Orally consumable preparation or food ingredient, comprising the combination remedy according to any one of claims 1 to 7.

15. Orally consumable preparation or food ingredient according to claim 14, which comprises the combination remedy according to any one of claims 1 to 7 in an amount of 1 to 80% by weight, based on the total weight of the orally consumable preparation or the food ingredient.

## Revendications

1. Composition combinée comprenant ou consistant en :
(a) un ou plusieurs premier(s) composant(s) de saturation, le ou les premier(s) composant(s) de saturation étant choisi(s) dans le groupe constitué par la nonivamide, la pellitorine, les dérivés d'alcool cinnamylique choisis dans le groupe constitué par le formiate de cinnamyle, l'acétate de cinnamyle, le propionate de cinnamyle, le butyrate de cinnamyle, l'isobutyrate de cinnamyle, l'isovalérianate de cinnamyle, le tiglinate de cinnamyle, le benzoate de cinnamyle, l'acétate de cinnamylphényle, et le cinnamate de cinnamyle, les dérivés d'amide d'acide cinnamique de formule générale 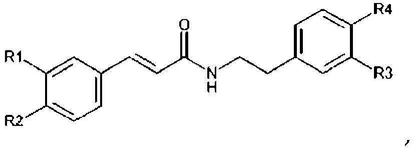 dans laquelle R1, R2, R3 et R4 représentent indépendamment un atome d'hydrogène ou un groupe méthoxy, des dérivés d'acides alcénoïques aromatiques choisis dans le groupe constitué par la dihydropiperlinguminine, la chingchenamide A, la piperdardine et le 7-(1,3-benzodioxol-5-yl)hepta-2,4-diénoate de méthyle, et leurs mélanges ;
(b) de l'arginine libre ajoutée ou ses stéréoisomères possibles et des mélanges de stéréoisomères ou ses sels de sodium, de calcium, de magnésium, de potassium ou d'ammonium ; et
éventuellement
(c) un ou plusieurs deuxièmes composants de saturation, lesdits un ou plusieurs deuxièmes composants de saturation étant choisis dans le groupe constitué par les protéines végétales ou animales et les hydrolysats de protéines ; et/ou
(d) une ou plusieurs substance(s) sensoriellement active(s).

2. Composition combinée selon la revendication 1, dans laquelle le ou les premier(s) composant(s) de saturation est/sont choisi(s) dans le groupe constitué par la nonivamide, la pellitorine, et leurs mélanges.

3. Composition combinée selon l'une quelconque des revendications précédentes, dans laquelle le ou les plusieurs deuxième(s) composant(s) de satiété sont une ou des protéine(s) choisie(s) dans le groupe constitué par les protéines de viande (mammifères, oiseaux, reptiles, amphibiens, poissons), de crustacés, de coquillages, de mollusques, d'insectes, d'œufs, de lait, en particulier de caséine et de lactosérum, d'algues, de céréales, en particulier de blé, d'orge, de colza, de tournesol, de riz, de pommes de terre, de maïs, de soja, de haricots, de pois, de lentilles, de lupins, d'arachides, de luzerne, de chanvre et d'autres protéines de plantes comestibles, en particulier des protéines à teneur intrinsèquement élevée en L-arginine.

4. Composition combinée selon l'une des revendications précédentes, dans laquelle le ou les plusieurs deuxième(s) composant(s) de satiété est/sont un ou plusieurs hydrolysat(s) de protéines choisi(s) dans le groupe constitué par l'hydrolysat partiel ou complet de protéines de viande (mammifères, oiseaux, reptiles, amphibiens, poissons), de crustacés, de coquillages, de mollusques, d'insectes, d'œufs, de lait, notamment de caséine et de lactosérum, d'algues, de céréales, notamment de blé, d'orge, de colza, de tournesol, de riz, de pommes de terre, de maïs, de soja, de haricots, de pois, de lentilles, de lupins, d'arachides, de luzerne, de chanvre et d'autres protéines issues de plantes comestibles, notamment de protéines à teneur intrinsèquement élevée en L-arginine.

5. Composition combinée selon l'une quelconque des revendications précédentes, dans laquelle la teneur totale en L-arginine dans la composition combinée selon l'invention comprenant les ingrédients (a) et (b) ou les ingrédients (a), (b) et (c) est d'au moins 0,1 g de L-arginine, de préférence d'au moins 0,5 g de L-arginine et plus préférablement d'au moins 1 g de L-arginine, dans une quantité pouvant être consommée de manière régulière (taille de portion), sur la base du poids total de la quantité pouvant être consommée de manière régulière (taille de portion) de la composition combinée.

6. Composition combinée selon l'une quelconque des revendications précédentes, qui comprend en outre :
(e) un support et/ou un adjuvant.

7. Composition combinée selon l'une quelconque des revendications précédentes, comprenant ou consistant en :
0,00001 % en poids (0,1 ppm) à 0,001 % en poids (10 ppm) des un ou plusieurs premier(s) composant(s) de saturation ;
5 à 20 % en poids d'arginine libre ajoutée ; et éventuellement
1 à 95 % en poids du ou des plusieurs deuxièmes composants de saturation ;
et/ou
0,00001 % en poids (0,1 ppm) à 1 % en poids de la ou des substances sensoriellement actives ;
dans chaque cas, par rapport au poids total de la composition combinée.

8. Composition combinée selon l'une quelconque des revendications 1 à 7, à usage médical.

9. Composition combinée selon l'une des revendications 1 à 7 pour une utilisation dans le traitement du surpoids et de l'obésité.

10. Composition combinée selon l'une des revendications 1 à 7 pour une utilisation dans la prévention des maladies cardiovasculaires, des accidents vasculaires cérébraux, du diabète ainsi que des lésions articulaires et de l'usure de la colonne vertébrale.

11. Composition combinée selon l'une des revendications 1 à 7 pour une utilisation non thérapeutique.

12. Utilisation de la composition combinée selon l'une des revendications 1 à 7 comme agent pour une application non thérapeutique
(i) réduction de l'appétit ; et/ou
(ii) procuration d'une sensation de satiété ; et/ou
(iii) réduction de l'apport énergétique ; et/ou
(iv) réduction du poids corporel.

13. Utilisation de la composition combinée selon l'une quelconque des revendications 1 à 7 pour la fabrication d'une préparation consommable par voie orale ou d'un ingrédient alimentaire.

14. Préparation consommable par voie orale ou ingrédient alimentaire comprenant la composition combinée selon l'une quelconque des revendications 1 à 7.

15. Préparation ou ingrédient alimentaire consommable par voie orale selon la revendication 14, qui comprend la composition combinée selon l'une quelconque des revendications 1 à 7 en une quantité de 1 à 80 % en poids, par rapport au poids total de la préparation ou de l'ingrédient alimentaire consommable par voie orale.
